(19) Europäisches Patentamt
European Patent Office
Office européen des brevets

(11) **EP 1 843 794 B1**

(12) **FASCICULE DE BREVET EUROPEEN**

(45) Date de publication et mention
de la délivrance du brevet:
**26.09.2012 Bulletin 2012/39**

(21) Numéro de dépôt: **06700552.0**

(22) Date de dépôt: **20.01.2006**

(51) Int Cl.:
*A61L 2/18* (2006.01)          *B01J 13/00* (2006.01)
*B01F 17/00* (2006.01)

(86) Numéro de dépôt international:
**PCT/IB2006/000151**

(87) Numéro de publication internationale:
**WO 2006/079911 (03.08.2006 Gazette 2006/31)**

(54) **PROCEDE DE CONTROLE DE LA CONTAMINATION MICROBIENNE DE SUSPENSIONS OU DISPERSIONS AQUEUSES MINERALES**

VERFAHREN ZUR KONTROLLE DER MIKROBIELLEN VERSCHMUTZUNG VON MINERALSUSPENSIONEN

METHOD FOR CONTROLLING MICROBIAL CONTAMINATION OF MINERAL SUSPENSIONS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **26.01.2005 FR 0500779**

(43) Date de publication de la demande:
**17.10.2007 Bulletin 2007/42**

(73) Titulaire: **OMYA DEVELOPMENT AG**
**4665 Oftringen (CH)**

(72) Inventeurs:
• **BURI, Matthias**
**CH-4852 Rothrist (CH)**
• **SCHWARZENTRUBER, Patrick**
**CH-8113 Boppelsen (CH)**
• **HUBSCHMID, Silvia**
**CH-4600 Olten (CH)**

(74) Mandataire: **Richebourg, Michel François**
**Cabinet Michel Richebourg**
**"Le Clos du Golf"**
**69 Rue Saint-Simon**
**42000 Saint Etienne (FR)**

(56) Documents cités:
**EP-A- 0 398 487          EP-A- 0 850 283**
**WO-A-01/85659          WO-A-02/13774**
**DE-A1- 19 811 742          DE-C1- 4 233 600**
**FR-A- 2 683 539          US-A- 3 659 708**
**US-A- 5 167 707          US-A1- 2001 008 877**
**US-B1- 6 756 437**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne en premier lieu un procédé de désinfection et/ou de conservation et/ou de réduction et/ou de contrôle de la contamination microbienne de dispersions aqueuses et/ou de suspensions aqueuses de matières minérales, et assurant une bonne stabilité en terme de viscosité Brookfield™ auxdites dispersions et/ou suspensions aqueuses de matières minérales.

Un autre objet de l'invention réside dans l'utilisation des suspensions et/ou des dispersions aqueuses de matières minérales, présentant une bonne stabilité en terme de viscosité Brookfield™ et possédant un nombre très réduit de germes microbiens et/ou dont on peut contrôler la concentration de germes microbiens au moyen du procédé selon l'invention,

Un autre objet de l'invention consiste en l'utilisation desdites suspensions et/ou dispersions aqueuses de matières minérales dans l'industrie minéralière, dans l'industrie du papier, de préférence dans la fabrication du papier, et/ou dans le couchage du papier, ainsi que dans le domaine de la fabrication des peintures aqueuses et notamment dans les laques et vernis.

Un dernier objet de l'invention réside dans les formulations minéralières, les formulations papetières et notamment les feuilles de papier et les sauces de couchage papetières, les peintures aqueuses, les laques et les vernis caractérisés en ce qu'ils contiennent lesdites suspensions et/ou dispersions aqueuses de matières minérales selon l'invention.

Un premier objet de l'invention est donc un procédé de désinfection et/ou de conservation et/ou de réduction et/ou de contrôle de la contamination microbienne de suspensions et/ou de dispersions aqueuses de matières minérales pour la protection face à la contamination microbienne et/ou le contrôle à dessein de la croissance d'un micro-organisme lors de la préparation desdites dispersions et/ou suspensions, de leur stockage, de leur transport et lors de leur modification et/ou traitement pendant un intervalle de temps réglable par l'utilisateur. De préférence, le procédé est utilisé dans les mines, dans l'industrie du papier, ainsi que dans l'industrie des vernis et peintures.

Le procédé a essentiellement pour but la réduction de la concentration et/ou l'évitement des biocides classiques, tels qu'entre autres spécifié dans la "XXXVI Empfehlung" vom BgVV (Bundesinstitut für gesundheitlichen Verbraucherschutz und Vetrinärmedizin, Deutschland) in "Kunststoffe im Lebensmittelverkehr" Carl Heymanns Verlag kg, Köln, Berlin, Bonn, München et dans le Code Fédéral 21 § 176.300, révision du 1er avril 2001. Il réduit de ce fait les risques de contamination et d'empoisonnement pour les humains et de dégradations de l'environnement lorsque de tels biocides étaient utilisés selon l'art antérieur, seuls, et en concentrations généralement élevées.

Un autre but est de créer un procédé qui comprenne un intervalle de temps, pouvant être choisi librement, pendant lequel le système doit agir.

Un autre but important est de ne pas influer ou bien, si cela devait être le cas, de façon positive, sur les propriétés des produits traités et/ou sur leur utilisation ultérieure.

Un autre but est de combiner un tel traitement aux étapes usuelles de fabrication de minéraux et/ou de pigments et/ou de charges, telles que notamment les étapes communes de dispersion et/ou de broyage dans l'eau desdites charges.

Un dernier but est de fournir un procédé qui n'altère pas la stabilité en terme de viscosité Brookfield™ des suspensions et des dispersions aqueuses de matières minérales ainsi obtenues.

Dans la présente demande, on désigne à travers le terme " microbes" tout organisme et/ou micro-organisme, aérobie ou anaérobie, à caractère bactérien tel que des germes bactériens, et notamment des germes bactériens mésophiles aérobies, tels que pseudomonas aeruginosa, salmonella enteritidis et escherichia coli, en tant que représentants gramme-négatifs, et bacillus subtilis, staphylococcus aureus, listeria monocytogenes et micrococcus luteus, en tant que représentants gramme-positifs, mais aussi des germes bactériens anaérobies et des germes bactériens réducteurs de sulfates anaérobies, tels que desulfovibrio desulfuricans, mais aussi des champignons, et notamment aspergillus niger, ainsi que des levures, et notamment saccharomyces cerevisiae.

On entend également, à travers l'expression de "désinfection et/ou conservation" le fait que de l'eau et/ou des solutions aqueuses et/ou des suspensions aqueuses et/ou des dispersions aqueuses contenant des matières minérales sont prémunies contre une attaque microbienne et/ou sont protégées du risque d'une infection microbienne, principalement par empêchement de la croissance et/ou par destruction desdits microbes.

Ces notions de désinfection et de conservation recouvrent donc ensemble les effets curateurs et protecteurs en terme de protection desdites suspensions et/ou dispersions aqueuses de matières minérales vis-à-vis d'une attaque microbienne.

Enfin, les termes "dispersions" et "suspensions" de matières minérales font référence dans la présente demande à une composition contenant de l'eau, des matières minérales dont la concentration en poids sec est supérieure ou égale à 0,1 % par rapport au poids total desdites dispersions et suspensions, ainsi qu'éventuellement d'autres additifs tels que notamment des agents dispersants, des agents d'aide au broyage et des agents anti-mousse.

Aujourd'hui, pour réaliser la désinfection et la protection de l'eau et/ou des solutions aqueuses et/ou des suspensions aqueuses et/ou des dispersions aqueuses contenant des matières minérales, l'homme du métier dispose de deux types de solutions, qu'il peut utiliser seules ou en combinaison : la mise en oeuvre de produits chimiques organiques dénommés sous le terme de biocides, ou le recours à des procédés de traitement ne faisant pas intervenir ces biocides. La Demanderesse va désormais présenter l'état de la technique relatif à ces deux voies, tout en soulignant les inconvénients que constituent l'ensemble de ces solutions actuelles.

Les dispersions et les suspensions aqueuses de minéraux et/ou de charges et/ou de pigments sont usuellement conservées au moyen de biocides qui peuvent être appliqués individuellement ou en combinaison. Les substances usuelles à effet biocide pour utilisation dans des suspensions aqueuses et/ou des dispersions aqueuses de matières minérales ainsi que dans les eaux de circuit industrielles sont entre autres énumérées dans le Code of Federal Regulations 21, §170 à §199, modifié en avril 2000, paragraphe 176.300, Slimicides. De telles substances sont également traitées dans l'ouvrage "Praxis der Sterilisation, Desinfektion-Konservierung" de Karl Heinz Wallhäusser, 5ème édition complètement remaniée, parue chez Georg Thieme Verlag, Stuttgart et dans le document "Microbicides for the protection of materials, a handbook by Wilfried Paulus" fist edition 1993, paru chez Chapman & Hall, 2-6 Boundary Row, London SE1 8HN. En outre, dans le "Code of Federal Regulation 21, §170 à §199, modifié en avril 2001, de telles substances à effets biocides sont décrites au paragraphe 176.170 et 176.300".

Parmi les formulations biocides largement répandues, certaines contiennent le 1,2 benzisothiazoline-3-on. L'inconvénient de telles formulations est ce que l'on appelle une "fenêtre pseudomonas", c'est-à-dire que la substance a un effet biocide contre une pluralité de bactéries mais présente cependant une efficacité moindre contre certaines bactéries, en l'occurrence les pseudomonas. En outre, cette substance entraîne une sensibilisation cutanée et, de ce fait, s'avère dangereuse pour l'utilisateur. Un autre inconvénient réside dans la stabilité dudit produit, de sorte que lors d'une application ultérieure, l'effet bactéricide du 1,2 benzisothiazoline-3-on n'est pas annulé et qu'il peut alors influer sur des produits alimentaires en traversant des substances d'emballage pour de tels produits et/ou des objets utilitaires pour des produits alimentaires. D'autre part, la mauvaise dégradabilité de ce composé et sa forte toxicité ont un effet destructeur sur l'environnement en cas de migration dudit produit à travers les emballages qui le contiennent, ou en cas de dégradation desdits emballages.

En outre, l'homme du métier peut également utiliser des mélanges de 5-chlore-2-méthyl-4-isothiazolinon et de 2-méthyle-4-isothiazolinon. Ici, l'inconvénient réside en ce que seul le 5-chlore-2-méthyl-4-isothiazolinon manifeste une efficacité suffisante par rapport aux bactéries ; or, cette substance est très instable à des valeurs de pH alcalines et à la chaleur et, de ce fait, perd rapidement son efficacité lorsqu'elle est utilisée dans des conditions de pH alcalin et/ou à des températures supérieures à 40°C. En outre, ces substances exercent également un effet sensibilisateur sur la peau.

On peut également utiliser des substances contenant du brome et, de manière plus générale, des combinaisons de produits halogénés. De telles combinaisons sont cependant indésirables dans de nombreux cas puisqu'elles peuvent dégrader l'environnement, en particulier dans le domaine du danger d'exposition à l'eau. Du fait de leur instabilité pour une valeur de pH neutre et alcaline, de tels biocides sont obligatoirement stabilisés à une valeur de pH acide et sont utilisés tels quels. Dans le cas d'un dosage effectué à une et/ou plusieurs reprises, des problèmes de compatibilité peuvent se produire avec des solutions de pigments réglées à un pH neutre et/ou alcalin. La stabilité de telles solutions peut de ce fait être dégradée en terme de viscosité Brookfield™. Tout particulièrement pour des dispersions ou des suspensions aqueuses fortement concentrées en matières minérales, notamment en carbonate de calcium et/ou en kaolin, on peut observer une augmentation de la viscosité et une formation d'agglomérats.

Il est également connu d'utiliser du glutardialdéhyde. Le glutardialdéhyde est instable au-dessus de températures de 40 à 45°C et se décompose ou forme des structures en anneau et perd ainsi son efficacité. De plus, le glutardialdéhyde fait actuellement l'objet de nombreuses études toxicologiques, portant notamment sur son caractère cancérigène : en effet, il n'est pas certain que ce produit soit dénué de tout risque pour l'homme au niveau mutagène. Si cet aspect n'est pas encore clairement démontré, en revanche, il est bien connu que le glutardialdéhyde peut engendrer des maladies respiratoires chroniques et des affections allergiques. Par conséquent, il représente un danger certain pour l'utilisateur. Un autre très grand groupe de biocides réside dans les produits qui se décomposent en donnant du formaldéhyde. De façon générale, ces produits ne sont pas très stables à la chaleur et se décomposent spontanément en formaldéhyde à des températures supérieures à 60°C. Or, le formaldéhyde est suspecté d'être cancérigène : selon une classification établie par l'Union Européenne, il est rangé dans la catégorie n°3 en tant que "substance préoccupante pour l'homme en raison d'effets cancérogènes possibles" et, du fait de sa volatilité élevée ($T^{eb}$ = -19,2°C pour le produit pur), il représente un risque important en cas d'utilisation. On utilise principalement comme dissociateur en formaldéhyde des formales-O et des formales-N, ainsi que de l'éthylène glycol-bis-hémiformal et du benzyl-bis-hémiformal. D'après l'ouvrage intitulé "Praxis der Sterilisation, Desinfektion-Konservierung, de Karl-Heinz Wallhäusser, 5ème édition complètement remaniée, parue chez Georg Thieme Verlag, Stuttgart, 1995, page 43", il est connu que les dérivés phénols sont utilisés en tant que principes actifs anti-microbiens.

Dans le document DE 100 27 588 A1, on propose 1' o-phénylphénol, et ses sels alcalins en tant qu'agent de conservation. Ceux-ci sont certes stables pour un pH alcalin et actifs contre la plupart des micro-organismes, mais, du fait de leur bonne stabilité chimique et thermique, il reste difficile de les désactiver. Or, il est parfois essentiel que leur effet anti bactérien ne soit pas permanent : il s'agit d'une exigence de la plus haute importance que l'on rencontre notamment dans le domaine papetier. Ainsi, le document WO 04 / 90148 décrit-il la synthèse enzymatique d'un polymère du type acrylamide, utilisé comme agent coagulant et/ou adhésif et/ou épaississant dans la fabrication du papier. Par ailleurs, le document CN 1 483 773 enseigne l'utilisation d'un composé enzymatique dans un procédé de désencrage du papier.

On peut encore citer le document JP 2004 169 243 qui décrit un procédé mettant en oeuvre une enzyme pour blanchir la pulpe utilisée dans la fabrication de papier. Par conséquent, ces documents démontrent bien l'importance que peuvent avoir certaines enzymes dans le domaine papetier : il est donc important de disposer d'un moyen de protection microbienne dont on peut contrôler l'activité, pour ne pas nuire à la présence desdites enzymes qui s'avèrent essentielles dans certains procédés papetiers. En outre, il est connu que l'o-phénylphénol présente aussi bien un effet curateur que protecteur : or, ces deux aspects sont d'égale importance pour l'homme du métier. On entend respectivement par effet curateur et protecteur les caractères d'un procédé ou d'une substance visant à assurer la protection respectivement contre une infection subséquente ou contre une infection s'étant déjà produite (tels que décrits dans le document "Wörterbuch der Mikrobiologie H. Weber, Gustav Fischer Verlag, Jena, Stuttgart, Lüber, Ulm", 1997, respectivement page 449 et page 321).

De plus, les procédés de dosage des substances microbicides pour la désinfection et la conservation de suspensions et/ou de dispersions aqueuses, présentent des inconvénients dans le domaine de la protection des personnes, de la stabilité à la chaleur et/ou de la dégradation de l'environnement : leur utilisation doit donc être évitée.

Une autre méthode pour assurer la désinfection et la conservation de l'eau et/ou des suspensions aqueuses et/ou des dispersions aqueuses contenant des matières minérales, réside dans la mise en oeuvre de procédés de traitement qui ne font pas intervenir de produits chimiques.

A ce titre, dans le domaine des produits alimentaires, d'une part, on stérilise et on conserve les substances à effet microbicide, en utilisant notamment la chaleur (par exemple procédé UHT). Une trop forte chaleur peut néanmoins conduire à une modification des produits à protéger et, de ce fait, n'est pas recommandable dans de nombreux cas. Les vitamines, par exemple, peuvent être détruites par des températures trop élevées.

Dans la littérature sont également décrits des procédés faisant usage de l'électrophorèse. Ici, on génère entre autres de l'hydrogène ou de l'oxygène à l'état naissant. Or, il est bien connu que l'hydrogène à l'état naissant, en particulier en présence d'oxygène, entraîne un risque d'explosion (gaz détonant).

En outre, il est connu de réaliser une stérilisation au moyen de rayons X. Les sources de rayons X peuvent cependant s'avérer dangereuses si on les manipule de façon non conforme, elles exigent un personnel formé spécialement et de ce fait représentent l'inconvénient d'être coûteuses et difficiles à mettre en oeuvre.

Par ailleurs, il est connu que l'ozone est utilisé comme agent de désinfection. L'ozone est cependant toxique et coûteux à fabriquer et, de ce fait, ne convient pas spécialement pour l'utilisation sur site. L'ozone peut également dégrader l'effet des dispersants, tels que les polyacrylates de sodium, ce qui, de nouveau, conduit à une augmentation indésirable de la viscosité de la suspension et/ou de la dispersion aqueuse à traiter.

On utilise également le rayonnement UV, en particulier le rayonnement UV-C pour la stérilisation. Le rayonnement UV est cependant dangereux. La lumière UV, par exemple, est utilisée dans la stérilisation de l'eau. Des substances troubles peuvent s'avérer être mal traitées par utilisation de rayonnement UV (phénomènes d'ombre).

La "Hochschule CH-8820 Wädenswil", Suisse, publie un procédé qui réduit les bactéries en utilisant de fortes impulsions électriques (procédé "High Electric Field Puls" et bulletin ASE/AES 3/01, page 44) : il s'agit ici aussi d'un procédé qui nécessite de lourdes modifications dans le procédé de fabrication de matières minérales pour qu'il soit mis en oeuvre.

En outre, dans l'ouvrage "J. Food Prot. Vol. 64 No. 10 2001, pages 1579 à 1583, Author-Department of Applied Chemistry, Kanagawa Institute of Technology, Atsugi, Japan", est décrit un procédé de désinfection des produits alimentaires en utilisant des coquilles de crabes calcinées. On cuit ici des coquilles à une température supérieure à 850 °C et le CaO produit est proposé comme agent désinfectant pour des produits alimentaires. On n'entre pas dans le détail des éventuels effets négatifs sur les produits traités. L'homme de l'art ne peut, à partir de ce document, obtenir quelque connaissance que ce soit sur l'influence exercée sur les suspensions ou dispersions aqueuses de pigments obtenues et sur la modification de leurs propriétés, tel que le comportement en viscosité. En outre, rien n'est dit au sujet d'une efficacité limitée dans le temps et aucun renvoi n'est donné à un autre traitement possible visant à créer un intervalle de temps pendant lequel le système doit agir.

De surcroît, dans le Brock-Biology of Microorganisms- (9ème édition), Madigan, M.T., et Martinko, J.M., et Perker, J., 2000, Upper Saddle River, (Prentice-Hall, Inc.), pages 154 à 155 à la figure 5.18 et dans "Allgemeine Mikrobiologie, (7ème édition), Schlegel, H.G., 1992, Georg Thieme Verlag, Stuttgart - New York, pages 194 à 196, il est mentionné que quelques micro-organismes tels que bacillus spezies peuvent également vivre dans un milieu extrêmement alcalin. Dans ce document, il n'existe aucune mention au fait que, en augmentant et en diminuant la valeur du pH, on peut obtenir une conservation temporaire de suspensions aqueuses de matières minérales sans altérer les propriétés physiques desdites suspensions en terme de viscosité. D'autre part, ce document qui tendrait à démontrer que certains micro-organismes peuvent subsister dans des conditions de pH alcalines n'incite pas l'homme du métier à mettre en oeuvre de telles conditions de pH alcalins, précisément en vue de protéger et/ou de désinfecter des suspensions aqueuses de matières minérales, ce qui est un objet de la présente invention. La Demanderesse tient à souligner que ni ce procédé, pas plus que les méthodes utilisant la chaleur, l'ozone, les rayons X ou UV, les impulsions électriques, ne permettent le contrôle de la croissance microbienne dans les suspensions de matières minérales à traiter. Comme il a déjà été indiqué, il s'agit d'une exigence importante pour l'homme du métier, notamment dans l'industrie papetière. Enfin, le document DE 19 811 742 décrit un procédé de traitement de l'eau pour la purifier en carbonate de calcium et en kaolin, en augmentant le pH à une valeur supérieure à 12, et préférentiellement supérieure à 12,6 - 12,8, par addition d'oxyde ou d'hydroxyde de calcium. Ce document, certes non situé dans le domaine technique particulier des procédés de traitement des suspensions aqueuses de matières minérales pour en éliminer les bactéries, ne peut être ignoré par l'homme du métier : il se rapporte en effet à un procédé de traitement plus général de telles suspensions de matières minérales, en vue de les purifier. Il enseigne que l'addition d'oxyde ou d'hydroxyde de calcium conduit dans ce cas à une floculation des matières minérales en suspension, ce qui est un effet non recherché dans le cadre du problème technique que la Demanderesse cherche à résoudre (la diminution ou le contrôle de la croissance microbienne). Or, de manière surprenante, grâce au procédé mis au point dans la présente demande, on parvient à résoudre le problème posé sans pour autant faire floculer les matières minérales en suspension et ce, éventuellement par addition d'oxyde ou d'hydroxyde de calcium. Aussi, de manière surprenante, le procédé selon l'invention ne conduit pas à une floculation des suspensions et des dispersions aqueuses de matières minérales auxquelles il est appliqué.

En définitive, l'usage de biocides tels que décrits dans l'art antérieur présente de nombreux inconvénients, en terme de dangerosité pour l'être humain, et/ou de dégradation de l'environnement.

D'autre part, les procédés actuellement mis en oeuvre pour décontaminer les suspensions aqueuses de matières minérales s'avèrent en général coûteux, difficiles à installer dans un procédé de fabrication desdites matières minérales, et non dénués eux aussi de dangers pour l'environnement et de risques pour l'être humain.

Par ailleurs, aucun des produits chimiques usuels et aucun des procédés de décontamination connu ne permet de contrôler l'évolution de la croissance, c'est-à-dire l'évolution de la division cellulaire des microbes et/ou le nombre total de microbes au cours du temps, dans lesdites suspensions aqueuses de matières minérales. Or, comme la Demanderesse l'a déjà indiqué, c'est une exigence fondamentale pour l'homme du métier, notamment dans le secteur de la fabrication du papier.

De plus, il est primordial de développer un procédé qui n'altère pas la stabilité, en terme de viscosité Brookfield™, des dispersions et/ou des suspensions aqueuses de matières minérales ainsi obtenues.

Ces problèmes sont entièrement résolus par l'invention qui consiste en un procédé de désinfection et/ou de conservation et/ou de réduction et/ou de contrôle de la contamination microbienne de dispersions et/ou de suspensions aqueuses de matières minérales, caractérisé en ce qu'il met en oeuvre :

a) au moins une étape d'augmentation de la concentration en ions OH⁻ desdites dispersions et/ou suspensions aqueuses, à une valeur supérieure ou égale à $1 \times 10^{-2}$ mole/1,
b) au moins une étape de dispersion et/ou de broyage desdites dispersions et/ou suspensions aqueuses, intervenant avant, pendant ou après l'étape a), mettant éventuellement en oeuvre au moins un agent dispersant et/ou au moins un agent d'aide au broyage,
c) éventuellement au moins une étape de diminution de la concentration en ions OH⁻ desdites dispersions et/ou desdites suspensions aqueuses, intervenant après l'étape a), à une valeur inférieure ou égale à $1 \times 10^{-2}$ mole/l,
d) éventuellement au moins une étape d'addition d'au moins une substance à effet microbicide et/ou de mise en oeuvre d'un procédé physique de décontamination, intervenant avant, pendant ou après l'étape a) et/ou b) et/ou c).

La Demanderesse souligne que, le procédé étant ainsi défini, les étapes a), b), c) et d) peuvent être répétées autant de fois que nécessaire, à la convenance de l'homme du métier qui saura adapter le procédé selon l'invention aux dispersions et/ou aux suspensions aqueuses de matières minérales qu'il envisage de traiter.

Ce procédé est donc caractérisé par une augmentation de la concentration en ions OH⁻ à l'aide d'un ou plusieurs donneurs d'ions OH⁻, comme des oxydes alcalins et/ou alcalino-terreux et/ou hydroxydes alcalins et/ou alcalino-terreux pour diminuer la vitesse de la division cellulaire biologique et/ou faire cesser la division cellulaire biologique et/ou détruire les microbes présents dans ladite dispersion et/ou suspension aqueuse.

Et, en cas de besoin, on réalise une diminution de la concentration en ions OH⁻ de la suspension et/ou de la dispersion aqueuse, à l'aide d'un ou plusieurs donneurs d'ions $H_3O^+$ faibles, moyennement forts ou forts, monovalents et/ou polyvalents, tel que notamment le $CO_2$ gazeux dissocié dans l'eau en acide carbonique, ceci permettant de rétablir la croissance naturelle des germes microbiens.

Ce procédé permet, tant dans la phase de limitation de la croissance des germes microbiens, que dans la phase de propagation desdits germes, d'éviter toute dégradation de la suspension et/ou de la dispersion aqueuse de matières minérales vis-à-vis de son application ultérieure, telle que, par exemple, une dégradation de son aptitude au stockage, de sa pompabilité et ou toute altération de son comportement rhéologique en terme de viscosité.

Un but important de l'invention est donc de simplifier la procédure de désinfection et/ou de conservation des suspensions et/ou des dispersions aqueuses de matières minérales en combinaison avec d'autres étapes de fabrication telles que notamment le broyage et/ou la dispersion desdites matières minérales, sans pour autant altérer la stabilité en terme de viscosité Brookfield™ desdites suspensions et/ou dispersions aqueuses de matières minérales.

Un autre but de l'invention est de fournir une suspension et/ou une dispersion aqueuse de matières minérales par un procédé permettant la désinfection et/ou la protection de ladite suspension et/ou de ladite dispersion aqueuse contre toute contamination microbienne et/ou attaque microbienne, tout en ménageant les humains, l'environnement et les ressources naturelles. Il faut en particulier veiller à ne pas utiliser inutilement des substances chimiques à risques, sachant que la combinaison du procédé selon l'invention, avec les quantités utilisées appropriées, de nocivité minimale et/ou plus faibles, de substances chimiques, telles que, par exemple, le o-phénylphénol et ses sels, peut constituer une forme de réalisation préférée. Le procédé doit pouvoir être applicable à des espèces aérobies et anaérobies.

Un autre but important de l'invention est de contrôler l'évolution de la croissance, c'est-à-dire l'évolution de la division cellulaire biologique et/ou le nombre total de micro-organismes au cours du temps, de manière à ne pas dépasser un nombre déterminé de microbes. En outre, l'effet microbicide peut être supprimé de manière simple, sans altérer la stabilité des suspensions et/ou des dispersions aqueuses traitées en terme de viscosité Brookfield™, sans restreindre leurs utilisations ultérieures notamment dans le secteur papetier, par la mise en oeuvre d'enzymes qui est parfaitement compatible avec le procédé selon l'invention.

Un autre but de cette invention concerne l'épuration et la désinfection des réservoirs de stockage, des récipients de transport ferroviaire et routier, tels que les réservoirs en béton et en acier, les wagons citernes ferroviaires, les citernes et les conteneurs. Les wagons citernes ferroviaires qui sont utilisés pour le transport des suspensions aqueuses de pigments contiennent des quantités résiduelles de pigments sous forme liquide et en partie concentrées par séchage. A leur retour, ils doivent être nettoyés et désinfectés pour éviter toute contamination d'un nouveau produit à charger. Il en va de même pour tout « récipient » de stockage et de transport, quels que soient sa taille et son volume. Ici, également, il est indispensable de procéder à l'annulation de l'effet microbicide « juste à temps » (« just in time ») pour ne pas exposer les humains, les animaux et l'environnement à un quelconque danger.

Le problème est résolu selon l'invention par le fait que l'on fournit un procédé qui, seul ou en combinaison avec d'autres procédés, tels que l'utilisation supplémentaire de substances à effet microbicide appropriées ou un procédé physique, tel que les impulsions à haute tension, le traitement thermique, permet une réduction et/ou une élimination et/ou un contrôle de la croissance de l'organisme microbien, procédé qui est d'une durée d'action limitée et peut être contrôlé. Le procédé possède donc autant des effets curateurs que protecteurs. Enfin, ledit procédé n'altère pas ou peu la stabilité, en terme de viscosité Brookfield™, desdites dispersions et/ou suspensions aqueuses de matières minérales ainsi traitées.

Un premier objet de l'invention est donc un procédé de désinfection et/ou de conservation et/ou de réduction et/ou de contrôle de la contamination microbienne de dispersions et/ou de suspensions aqueuses de matières minérales, caractérisé en ce qu'il met en oeuvre :

a) au moins une étape d'augmentation de la concentration en ions OH⁻ desdites dispersions et/ou desdites suspensions aqueuses, à une valeur supérieure ou égale à $1 \times 10^{-2}$ mole/l,
b) au moins une étape de dispersion et/ou de broyage desdites dispersions et/ou suspensions aqueuses, intervenant avant, pendant ou après l'étape a), mettant éventuellement en oeuvre au moins un agent dispersant et/ou au moins un agent d'aide au broyage,
c) éventuellement au moins une étape de diminution de la concentration en ions OH⁻ desdites dispersions et/ou desdites suspensions aqueuses, intervenant après l'étape a), à une valeur inférieure ou égale à $1 \times 10^{-2}$ mole/l,
d) éventuellement au moins une étape d'addition d'au moins une substance à effet microbicide et/ou de mise en oeuvre d'un procédé physique de décontamination, intervenant avant pendant ou après l'étape a), et/ou b), et/ou c).

Ce procédé est caractérisé en ce que la valeur de la concentration en ions OH relative à l'étape a) est préférentiellement supérieure ou égale à $2 \times 10^{-2}$ mole/l.

Ce procédé est également caractérisé en ce qu'on réalise l'augmentation de la concentration en ions OH⁻, relative à l'étape a), à l'aide d'un ou plusieurs donneurs d'ions OH⁻, comme des oxydes alcalins et/ou alcalino-terreux et/ou des hydroxydes alcalins et/ou alcalino-terreux.

Ce procédé est aussi caractérisé en ce que la valeur de la concentration en ions OH⁻ relative à l'étape c) est préférentiellement inférieure ou égale à $1 \times 10^{-3}$ mole/l, et très préférentiellement inférieure ou égale à $1 \times 10^{-4}$ mole/l.

Ce procédé est également caractérisé en ce qu'on réalise la diminution de la concentration en ions OH⁻, relative à l'étape éventuelle c), à l'aide d'un ou plusieurs donneurs d'ions $H_3O^+$ faibles, moyennement forts ou forts, monovalents et/ou polyvalents, tel que notamment le $CO_2$ gazeux dissocié dans l'eau en acide carbonique.

Ce procédé est également caractérisé en ce que l'étape éventuelle d) d'addition d'au moins une substance à effet microbicide et/ou de mise en oeuvre d'un procédé physique de décontamination microbicide mette en oeuvre au moins un biocide et notamment le o-phénylphénol et/ou ses sels ou bien leurs mélanges, et/ou au moins un produit contenant un germe destructeur des germes microbiens, préférablement des germes pseudomonas, plus préférablement des germes pseudomonas aeruginosa, et en ce que ce germe destructeur soit de la famille Bdellovibrio, et soit très préférentiellement le germe Bdellovibrio bacteriovorus.

Ce procédé est également caractérisé en ce que l'étape éventuelle d) d'addition d'au moins une substance à effet microbicide et/ou de mise en oeuvre d'un procédé physique de décontamination microbicide mette en oeuvre au moins un procédé physique, tel que préférentiellement les procédés basés sur une augmentation de la température.

Dans une variante de ce procédé correspondant à la réalisation de l'étape c), ledit procédé est caractérisé en ce que l'étape c) intervient préférentiellement entre une semaine et un mois après l'étape a).

Selon cette variante, il n'est alors pas mis en oeuvre de substance à effet microbicide dans les dispersions et/ou les suspensions aqueuses de matières minérales à traiter,

Ce procédé est aussi caractérisé en ce qu'il peut être mis en oeuvre en discontinu, en semi-continu ou en continu, selon la terminologie bien connue par l'homme du métier.

L'utilisation de ce procédé exerce des effets curateurs et/ou protecteurs vis-à-vis des eaux et/ou des dispersions et/ou des suspensions aqueuses de matières minérales à traiter.

Ce procédé est aussi caractérisé en ce que les matières minérales qu'il met en oeuvre sont choisies parmi les carbonates de calcium naturels : marbre, calcaire, dolomite et leurs mélanges, leurs mélanges avec d'autres minéraux, comme les mélanges talc-carbonate de calcium, carbonate de calcium-kaolin, ou encore les mélanges de carbonate de calcium avec le trihydroxyde d'aluminium ou le trioxyde d'aluminium, ou encore les mélanges avec des fibres synthétiques ou naturelles ou encore les co-structures des minéraux comme les co-structures talc-carbonate de calcium ou talc-dioxyde de titane, ou leurs mélanges, et/ou des carbonates de calcium contenant de la dolomite, ainsi que des carbonates de calcium fabriqués de façon synthétique par précipitation et/ou des précipités de carbonate de calcium avec d'autres minéraux. De manière préférentielle, ces matières minérales sont marbre, la calcite, la craie et leurs mélanges.

Ce procédé est enfin caractérisé en ce qu'il est mis en oeuvre dans les domaines de l'industrie minéralière, et notamment dans les réservoirs de stockage, les récipients de transport ferroviaire et routier, tels que les réservoirs en béton et en acier, les wagons citernes ferroviaires, les citernes et les conteneurs, dans l'industrie du papier, de préférence dans la fabrication du papier, et/ou dans le couchage du papier, ainsi que dans le domaine de la fabrication des peintures aqueuses et en outre dans les laques et vernis.

Un autre objet de l'invention réside dans l'utilisation des dispersions et/ou suspensions aqueuses de matières minérales obtenues par le procédé selon l'invention.

Ces dispersions et/ou suspensions sont aussi caractérisées en ce qu'elles contiennent des matières minérales choisies parmi les carbonates de calcium naturels, notamment du marbre, du calcaire, de la dolomite et leurs mélanges, leurs mélanges avec d'autres minéraux, comme les mélanges talc-carbonate de calcium, carbonate de calcium-kaolin, ou encore les mélanges de carbonate de calcium avec le trihydroxyde d'aluminium ou le trioxyde d'aluminium, ou encore les mélanges avec des fibres synthétiques ou naturelles ou encore les co-structures des minéraux comme les co-structures talc-carbonate de calcium ou talc-dioxyde de titane, ou leurs mélanges, et/ou des carbonates de calcium contenant de la dolomite, ainsi que des carbonates de calcium fabriqués de façon synthétique par précipitation et/ou des précipités de carbonate de calcium avec d'autres minéraux. De manière préférentielle, ces matières minérales sont choisies parmi du carbonate de calcium naturel et/ou précipité, et très préférentiellement sont choisies parmi des carbonates de calcium naturels et notamment parmi le marbre, la calcite, la craie et leurs mélanges.

Dans une première variante où l'étape c) du procédé selon l'invention n'est pas mise en oeuvre, cesdites dispersions et/ou suspensions sont caractérisées :

a) en ce qu'elles présentent une concentration en ions $OH^-$ supérieure ou égale à $1 \times 10^{-2}$ mole/l, préférentiellement supérieure ou égale à $2 \times 10^{-2}$ mole/l,

b) en ce qu'elles présentent une concentration en microbes inférieure ou égale à 100 microbes /gramme, et préférentiellement inférieure ou égale à 10 microbes /gramme,

c) et en ce qu'elles contiennent :

1. des matières minérales,
2. de l'eau,
3. éventuellement au moins un agent dispersant et/ou au moins un agent d'aide au broyage,
4. éventuellement au moins un agent anti-mousse,
5. éventuellement au moins un agent microbicide.

Selon cette variante, ces dispersions et/ou suspensions sont aussi caractérisées en ce qu'elles contiennent :

1. de 0,1 % à 85 % en poids sec de matières minérales,
2. de 15 % à 99,9 % en poids d'eau,
3. de 0 % à 5 % en poids sec d'au moins un agent dispersant et/ou d'au moins un agent d'aide au broyage,
4. de 0 % à 5 % en poids sec d'au moins un agent anti mousse,
5. de 0 % à 5 % en poids sec d'au moins un agent microbicide,

par rapport au poids total desdites dispersions et/ou suspensions.

Ces dispersions et/ou suspensions aqueuses sont aussi caractérisées en ce que la substance à effet microbicide est choisi parmi le o-phénylphénol, ses sels ou bien leurs mélanges, et/ou au moins un produit contenant un germe destructeur des germes microbiens, préférablement des germes pseudomonas, plus préférablement des germes pseudomonas aeruginosa, et en ce que ce germe destructeur soit de la famille Bdellovibrio, et soit très préférentiellement le germe Bdellovibrio bacteriovorus.

Toujours selon cette variante, et lorsqu'est mis en oeuvre selon l'étape d) un procédé basé sur l'augmentation de la température, ces dispersions et/ou suspensions aqueuses sont aussi caractérisées en ce que la concentration en microbes est inférieure à 10 microbes /gramme.

Dans une deuxième variante où l'étape c) du procédé selon l'invention est mise en oeuvre et où il n'est pas mis en oeuvre de substance à effet microbicide selon l'étape d), cesdites dispersions et/ou suspensions aqueuses sont caractérisées :

a) en ce qu'elles présentent une concentration en ions OH$^-$ inférieure ou égale à $1 \times 10^{-2}$ mole/l, préférentiellement inférieure ou égale à $1 \times 10^{-3}$ mole/l, très préférentiellement inférieure ou égale à $1 \times 10^{-4}$ mole/l,
b) en ce qu'elles présentent une concentration en microbes inférieure ou égale à 100 microbes /gramme, et préférentiellement inférieure ou égale à 10 microbes /gramme,
c) et en ce qu'elles contiennent :

1. des matières minérales,
2. de l'eau,
3. au moins un agent dispersant et/ou au moins d'un agent d'aide au broyage,
4. et éventuellement au moins un agent anti mousse,

Selon cette variante, les dispersions et/ou suspensions selon l'invention sont aussi caractérisées en ce qu'elles contiennent :

1. de 0,1 % à 85 % en poids sec de matières minérales,
2. de 10 % à 99, 89 % en poids d'eau,
3. de 0,01 % à 5 % en poids sec d'au moins un agent dispersant et/ou d'au moins un agent d'aide au broyage,
4. de 0 % à 5 % en poids sec d'au moins un agent anti mousse,

par rapport au poids total desdites dispersions et/ou suspensions.

Selon cette variante, l'agent anti-mousse est notamment choisi parmi des composés siloxanes, des esters d'acides gras et leurs mélanges.

Toujours selon cette variante, et lorsqu'est mis en oeuvre selon l'étape d) un procédé basé sur l'augmentation de la température, ces dispersions et/ou suspensions aqueuses sont aussi caractérisées en ce que la concentration en microbes est inférieure ou égale à 10 microbes / gramme.

Un autre objet de l'invention est l'utilisation de ces suspensions et/ou dispersions de matières minérales dans les domaines de l'industrie minéralière, dans l'industrie du papier, de préférence dans la fabrication du papier, et/ou dans le couchage du papier, ainsi que dans le domaine de la fabrication des peintures aqueuses et en outre dans les laques et vernis.

La présente invention est décrite plus en détails ci-après à l'aide d'exemples de réalisation et d'exemples comparatifs. L'invention n'est cependant pas limitée aux exemples suivants. L'homme de l'art est en mesure, sans déployer d'activité inventive, à l'aide de la présente description, en liaison avec les revendications, de formuler d'autres exemples et de trouver d'autres domaines d'application.

**EXEMPLES**

Remarques générales concernant la manière de procéder.

Les méthodes usuelles de détermination des germes dans l'industrie des produits alimentaires et dans l'industrie des papiers et des pigments sont par exemple décrites dans le manuel suisse sur les produits alimentaires, chapitre 56, paragraphe 7.01, édition 1985, révision 1988, intitulé "Bestimmung von aeroben Bakterien und Keime" et dans le manuel suisse des produits alimentaires, chapitre 56, paragraphe 7.22, édition 1985, révision 1988, intitulé "Bestimmung von Pilzen". Usuellement, le temps d'incubation avant de pouvoir effectuer une détermination est chaque fois d'environ 48 heures. On applique un temps d'incubation de 5 jours afin de déceler la présence des spores.

C'est la société Microbial Systems Ltd qui a développé le dispositif et le procédé d'analyse de particules commercialisés sous le nom de Cellfacts™ R. Des informations supplémentaires à ce sujet se trouvent dans le journal intitulé Labor flash 9/96, offrant un service lecteur pour le laboratoire et la recherche, Ott Verlag + Druck AG, Ch-3607 Thun, Suisse. Ces dispositifs permettent de déterminer, éventuellement par extrapolation, la concentration en bactéries dans un échantillon, en tant que particules présentes dans un champ électrique. Le dispositif en question ainsi que la méthode de mesure et les calculs correspondants sont détaillés dans le brevet européen EP 1 149 172.

Les suspensions de pigments utilisées dans les exemples ont été produites par broyage et/ou dispersion en présence de polyacrylates de sodium. La masse de l'échantillon initial était de 5 kg. On a utilisé, un broyeur à boule de type Dynomill, de contenance égale à 2 litres, disposant d'un agitateur ayant un disque denté d'un diamètre de 50 mm. On a mis en oeuvre comme corps broyant des perles de verre d'un diamètre de 2 mm et des perles de silicate de zircon d'un diamètre de 0,5 à 2 mm, mais également d'autres types de billes de broyage telles que notamment de la porcelaine, du silicate de zirconium, des oxydes de zirconium comme de la badeleite, et leurs mélanges, et/ou des oxydes d'aluminium ou des agents de broyage autogènes.

Les suspensions et/ou dispersions aqueuses de matières minérales ont été stérilisées pendant une heure à 141 °C dans des autoclaves pour l'examen des effets protecteurs du procédé selon l'invention.

Les suspensions et/ou les dispersions ont été incubées pendant une semaine à 32°C dans une étuve à incubation, puis à nouveau mélangées avec la quantité et le type correspondants de bactéries testées, pour l'examen des effets curateurs du procédé selon l'invention.

A certains intervalles de temps, on a procédé à une quantification des germes selon la méthode "Bestimmung von aeroben mesophilen Keimen", Schweizerisches Lebensmittelbuch, chapitre 56, paragraphe 7.01, édition 1985, révision 1988.

Les valeurs de la concentration molaire en ions OH⁻ ont toujours été déterminées à une température de 22°C (la constante de dissociation de l'eau, pKw, étant alors égale à 14)

$$K = \frac{[C_{H3O+}] \times [C_{OH-}]}{[C_{H2O}]^2}$$

Pour l'eau à 22°C, dans laquelle $C_{H3O}+ = C_{OH^-} = 10^{-7}$ M, on a : K l'eau (Kw) (22°C) = $10^{-14}$ M².

Or, la constante de dissociation de l'eau, $pK^w$, est fonction de la température. Ainsi, une valeur de pH de 10 mesurée à 22 °C correspond à une concentration en ions OH⁻ qui conduirait à une valeur de pH égale à 11 si elle était mesurée à 100 °C.

Par conséquent, et afin de prendre en compte l'influence de la température, on a utilisé la table suivante pour fixer les valeurs de la constante de dissociation de l'eau :

| Température[°C] | $K^w$ [M²] | $pK_W = -\log^{10} K^w$ |
|---|---|---|
| 0 | $0,13 \times 10^{-14}$ | 14,89 |
| 10 | $0,36 \times 10^{-14}$ | 14,45 |
| 16 | $0,63 \times 10^{-14}$ | 14,20 |
| 20 | $0,86 \times 10^{-14}$ | 14,07 |
| 22 | $1,00 \times 10^{-14}$ | 14,00 |
| 30 | $1,89 \times 10^{-14}$ | 13,73 |
| 50 | $5,60 \times 10^{-14}$ | 13,25 |
| 100 | $74,00 \times 10^{-14}$ | 12,13 |

D'autre part, dans toute la suite de la présente demande, on indique que l'expression viscosité Brookfield™ fait référence à la viscosité Brookfield™ mesurée sur un viscosimètre du même nom et de type RVT, à la vitesse de 100 tours / minute, en utilisant le module n°3.

**Exemple 1**

Cet exemple a pour but d'illustrer le procédé selon l'invention, dans son mode curateur, appliqué à une suspension aqueuse de matière minérale qui est du carbonate de calcium.

Il a également pour but d'illustrer que le procédé selon l'invention permet de contrôler l'évolution de la croissance de germes microbiens dans une telle suspension, sans altérer sa stabilité de manière significative.

*Suspension de pigments :*

On a préparé une suspension aqueuse à 78,3 % en poids de marbre naturel (dont 90% en poids des particules ont un diamètre inférieur à 2 $\mu$m, et 65 % en poids des particules ont un diamètre inférieur à 1 $\mu$m) obtenu par broyage, en utilisant 0,65 % en poids sec d'un polyacrylate neutralisé par un mélange sodium/magnésium du commerce, par rapport au poids sec de matières minérales.

La valeur du pH de la suspension après le broyage était de 9,7 mesurée à 20°C.

On a préparé chaque fois 2 échantillons d'un kilogramme de la suspension de pigments.

*Suspension microbienne*

On a réalisé un mélange de 7 types de microbes différents, gramme-négatives, principalement composées à partir de la famille des pseudomonas (en majorité de pseudomonas aeruginosa), isolées à partir d'une suspension de carbonate de calcium ayant germé naturellement, venant d'Autriche.

Les 7 différentes variétés de microbes ont pu être identifiées grâce au test API™ bien connu de l'homme du métier, et mis au point par la société BIOMERIEUX™.

Dans cette suspension, la concentration en germes microbiens est de 5 x $10^6$ germes/ml.

*Echantillon 1*

Le 1er échantillon correspond à 1 kg de ladite suspension de pigments qui a été mélangée à 0,025 moles d'ions OH- par addition d'hydroxyde de soude, sous bonne agitation. (NaOH ayant été ajouté en tant que solution à 2,5 Molaire).

La viscosité Brookfield™ immédiatement après addition d'hydroxyde de soude était de 308 mPa.s.

*Echantillon 2*

Le 2ème échantillon a servi d'échantillon comparatif par rapport à l'état de la technique, et correspond à 1 kg de la suspension de pigments décrite au début de l'exemple 1, sans addition de la solution donneurs d'ions OH-.

La viscosité Brookfield™ était de 389 mPa.s.

Les deux échantillons ont ensuite été mélangés à 10 ml de suspension microbienne puis incubés chaque fois pendant 24 heures à 30°C dans une étuve d'incubation : dans la suite de la demande, on désigne cette action sous le terme d'exposition. Pour chaque exemple, les échantillons sont exposés à la même suspension bactérienne.

On a alors mesuré pour chaque échantillon la concentration en germes (en nombre/ml), les valeurs de la concentration en ions OH- (en mole/l), ainsi que la viscosité Brookfield™ (mPa.s).

Ces données figurent dans les tableaux 1 et 2.

**Tableau 1**

| | Concentration en OH- (mole/l) | | Concentration en germes (nombre/ml) | |
|---|---|---|---|---|
| | Echantillon 1 | Echantillon 2 | Echantillon 1 | Echantillon 2 |
| Immédiatement avant la 1ère exposition | 1,6 x $10^{-2}$ | 5 x $10^{-5}$ | <$10^2$ | <$10^2$ |
| Immédiatement après la 1ère exposition | 1,6 x $10^{-2}$ | 5 x $10^{-5}$ | <$10^2$ | 1 x $10^5$ |
| Mesure 3 jours après la 1ère exposition, puis 2ème exposition | 1,6 x $10^{-2}$ | 5 x $10^{-5}$ | <$10^2$ | 1 x $10^5$ |
| 4 jours après la 2ème exposition | 1,6 x $10^{-2}$ | 5 x $10^{-5}$ | <$10^2$ | 4 x $10^7$ |

Ces résultats démontrent l'effet protecteur du traitement selon l'invention sur l'échantillon 1 : il n'y a pas eu augmentation du nombre de microbes.

**Tableau 2**

| | Concentration en OH⁻ (mole/l) | | Viscosité Brookfied™ (mPa.s) | |
|---|---|---|---|---|
| | Echantillon 1 | Echantillon 2 | Echantillon 1 | Echantillon 2 |
| Immédiatement avant la 1ère exposition | $1,6 \times 10^{-2}$ | $5 \times 10^{-5}$ | 310 | 390 |
| 1 jour après la 1ère exposition | $1,6 \times 10^{-2}$ | $5 \times 10^{-5}$ | 518 | 463 |
| 5 jours après la 1ère exposition : 2ème exposition | $1,6 \times 10^{-2}$ | $5 \times 10^{-5}$ | 804 | 676 |
| 4 jours après la 2ème exposition | $1,6 \times 10^{-2}$ | $5 \times 10^{-5}$ | 661 | 560 |
| 26 jours après la 1ère exposition | $1,6 \times 10^{-2}$ | $5 \times 10^{-5}$ | 907 | 790 |
| 26 jours après la 1ère exposition et après 5 minutes d'agitation intense | $1,6 \times 10^{-2}$ | $5 \times 10^{-5}$ | 339 | 430 |

De plus, la viscosité Brookfield™ de l'échantillon selon l'invention n'est pas altérée : elle évolue de manière similaire à celle de l'échantillon non traité.

Enfin, les viscosités Brookfield™ à 26 jours mesurées après agitation sont très proches des viscosités Brookfield™ initiales : le traitement selon l'invention n'altère donc pas la stabilité des suspensions aqueuses de matières minérales en terme de viscosité Brookfield™.

Après 26 jours, une partie de l'échantillon 1 selon l'invention ayant subi les précédentes expositions, est traitée par introduction de $CO_2$ gazeux, de manière à ramener la concentration en ions OH⁻ à une valeur de $5 \times 10^{-5}$ mole/l.

Cet instant correspond à l'instant T = 0 pour ce nouvel échantillon.

Cette partie de l'échantillon 1, désormais appelée échantillon 1-2, et représentant l'invention, va subir un certain nombre d'expositions supplémentaires.

On va alors réaliser pour l'échantillon 1-2 les mesures de la concentration en ions OH⁻, du nombre de germes microbiens, et de la viscosité Brookfield™.

)Les résultats apparaissent dans les tableaux 3 et 4.

**Tableau 3 (échantillon 1-2)**

| | Concentration en OH⁻ (mole/l) | Concentration en germes (nombre/ml) |
|---|---|---|
| Immédiatement avant la 1ère exposition | $5 \times 10^{-5}$ | $< 10^2$ |
| Immédiatement après la 1ère exposition | $6 \times 10^{-5}$ | $2 \times 10^6$ |

**Tableau 4 (échantillon 1-2)**

| | Concentration en OH⁻ (mole/l) | Viscosité Brookfield™ (mPa.s) |
|---|---|---|
| Immédiatement avant la 1ère exposition | $5 \times 10^{-5}$ | 238 |
| 1 jour après la 1ère exposition | $6 \times 10^{-5}$ | 580 |
| 5 jours après la 1ère exposition : 2ème exposition | $1 \times 10^{-4}$ | 790 |
| 12 jours après la 1ère exposition | $8 \times 10^{-5}$ | 648 |
| 26 jours après la 1ère exposition | $8 \times 10^{-5}$ | 998 |
| 26 jours après la 1ère exposition et après 5 minutes d'agitation intense | $1 \times 10^{-5}$ | 358 |

La viscosité Brookfield™ sans agitation de l'échantillon 1-2 selon l'invention n'augmente que faiblement sur la durée du stockage par rapport à l'échantillon comparatif. La stabilité n'est pas dégradée. La viscosité Brookfield™ à l'état agité après 26 jours est à peu près identique à la viscosité Brookfield™ initiale avant le traitement selon l'invention : la stabilité de l'échantillon selon l'invention n'est donc pas altérée en terme de viscosité Brookfield™.

Après 26 jours, une autre partie de l'échantillon 1 selon l'invention ayant subi les premières expositions telles que décrites au début de cet exemple, est traitée par introduction d'acide nitrique, de manière à ramener la concentration en ions OH$^-$ à une valeur égale à 8 x 10$^{-5}$ mole/l.

Cet instant correspond à l'instant T = 0 pour ce nouvel échantillon.

Cette partie de l'échantillon 1, désormais appelée échantillon 1-3, et représentant l'invention, va alors subir une nouvelle exposition.

On va alors réaliser pour l'échantillon 1-3 les mesures de la concentration en ions OH$^-$, et du nombre de germes microbiens.

Les résultats apparaissent dans le tableau 5.

### Tableau 5 (échantillon 1-3)

| | Concentration en OH$^-$ (mole/l) | Concentration en germes (nombre/ml) |
|---|---|---|
| Immédiatement avant la 1$^{ère}$ exposition | 8 x 10$^{-5}$ | < 10$^2$ |
| Immédiatement après la 1$^{ère}$ exposition | 1 x 10$^{-4}$ | 2 x 10$^5$ |

L'effet d'inhibition a été de nouveau supprimé par addition d'ions H$_3$O$^+$ grâce à l'ajout d'acide citrique, c'est-à-dire grâce au procédé selon l'invention.

Après 26 jours, une autre partie de l'échantillon 1 selon l'invention ayant subi les premières expositions telles que décrites au début de cet exemple, est traitée par introduction d'acide phosphorique, de manière à ramener la concentration en ions OH$^-$ à une valeur égale à 2,5 x 10$^{-5}$ mole/l.

Cet instant correspond à l'instant T = 0 pour ce nouvel échantillon.

Cette partie de l'échantillon 1, désormais appelée échantillon 1-4, et représentant l'invention, va alors subir une nouvelle exposition.

On va alors réaliser pour l'échantillon 1-4 les mesures de la concentration en ions OH$^-$, et du nombre de germes microbiens.

Les résultats apparaissent dans le tableau 6.

### Tableau 6 (échantillon 1-4)

| | Concentration en OH$^-$ (mole/l) | Concentration en germes (nombre/ml) |
|---|---|---|
| Immédiatement avant la 1$^{ère}$ exposition | 2,5 x 10$^{-5}$ | < 10$^2$ |
| Exposition | 2,5 x 10$^{-5}$ | > 10$^6$ |

L'effet d'inhibition a été de nouveau supprimé par addition d'ions H$_3$O$^+$ par ajout d'acide phosphorique, c'est-à-dire grâce au procédé selon l'invention.

**Exemple 2**

Cet exemple a pour but d'illustrer le procédé selon l'invention, dans son mode curateur et protecteur, appliqué à une suspension aqueuse de matière minérale qui est du carbonate de calcium.

Il a également pour but d'illustrer que le procédé selon l'invention permet de contrôler l'évolution de la croissance de germes microbiens dans une telle suspension, sans altérer sa stabilité en terme de viscosité Brookfield™.

*Suspension de pigments*

On a préparé une suspension aqueuse à 78,3 % en poids de marbre naturel (dont 90 % en poids des particules ont un diamètre inférieur à 2 $\mu$m, et 65 % en poids des particules ont un diamètre inférieur à 1 $\mu$m), obtenu par broyage en utilisant 0,65 % en poids sec par rapport au poids sec de matières minérales d'un polyacrylate neutralisé par un mélange de sodium et de magnésium du commerce. La valeur de pH de la suspension après le broyage était de 9,7 mesurée à 20°C.

2 échantillons de 1 kg ont été préparés d'après la suspension de pigment.

*Suspension microbienne*

On a préparé un mélange de 7 types de bactéries gramme-négatives différentes, principalement composé d'après la famille des pseudo-monades (majoritairement du pseudomonas aeruginosa), isolé à partir d'une boue de carbonate de calcium ensemencée naturellement par des germes, provenant d'Autriche.

Les 7 différentes variétés de microbes ont pu être identifiées grâce au test API™ bien connu de l'homme du métier, et mis au point par la société BIOMERIEUX™.

La concentration en germes microbiens de cette suspension est de $5 \times 10^6$ germes/ml.

*Echantillon 3*

Cet échantillon sert à illustrer le traitement selon l'invention, dans son mode protecteur.

Cet échantillon correspond à 1 kg de ladite suspension de pigments qui a été mélangée sous bonne agitation, à une solution de $Ca(OH)_2$ broyé (le diamètre moyen des particules étant égal à 2 $\mu$m) contenant $2,6 \times 10^{-2}$ mole/l d'ions $OH^-$. La viscosité Brookfield™ immédiatement après la précédente addition était de 357 mPa.s. Cet échantillon selon l'invention a été ensuite mélangé plusieurs fois à 10 ml de suspension microbienne et ensuite incubé dans une étuve d'incubation, pendant 24 heures à 30°C

L'échantillon 3 a été soumis à un certain nombre d'expositions et on a également mesuré les valeurs de la concentration en ions $OH^-$ (mol/l), de la concentration en germes microbiens (nombre/gramme) et de la viscosité Brookfield™ (mPa.s).

Les résultats correspondants apparaissent dans les tableaux 7 et 8.

**Tableau 7 (échantillon 3)**

|  | Concentration en $OH^-$ (mole/l) | Concentration en germes (nombre/ml) |
|---|---|---|
| Immédiatement avant la 1ère exposition | $2 \times 10^{-2}$ | $< 10^2$ |
| Immédiatement après la 1ère exposition | $2 \times 10^{-2}$ | $< 10^2$ |
| 3 jours après la 1ère exposition : 2ème exposition | $2 \times 10^{-2}$ | $< 10^2$ |
| 4 jours après la 2ème exposition | $2 \times 10^{-2}$ | $< 10^2$ |

Ces résultats démontrent l'effet protecteur du traitement selon l'invention sur l'échantillon 3 : il n'y a pas eu augmentation du nombre de germes microbiens.

**Tableau 8 (échantillon 3)**

|  | Concentration en $OH^-$ (mole/l) | Viscosité Brookfield™ (mPa.s) |
|---|---|---|
| Immédiatement avant la 1ère exposition | $2 \times 10^{-2}$ | 357 |
| 1 jour après la 1ère exposition | $2 \times 10^{-2}$ | 909 |
| 5 jours après la 1ère exposition : 2ème exposition | $2 \times 10^{-2}$ | 888 |
| 4 jours après la 2ème exposition | $2 \times 10^{-2}$ | 747 |
| 26 jours après la 1ère exposition | $2 \times 10^{-2}$ | 999 |
| 26 jours après la 1ème exposition et après 5 minutes d'agitation intense | $2 \times 10^{-2}$ | 420 |

De plus, la viscosité Brookfield™ de l'échantillon selon l'invention n'est pas altérée : elle évolue de manière similaire à celle de l'échantillon non traité, représenté par l'échantillon 2.

Enfin, les viscosités Brookfield™ à 26 jours mesurées après agitation sont très proches des viscosités Brookfield™ initiales : le traitement selon l'invention permet donc de retrouver la viscosité Brookfield™.

Après 26 jours, une partie de l'échantillon 3 selon l'invention ayant subi les précédentes expositions, est traitée par introduction de $CO_2$ gazeux, de manière à ramener la concentration en ions $OH^-$ à une valeur égale à $5 \times 10^{-5}$ mole/l.

Cet instant correspond à l'instant T = 0 pour ce nouvel échantillon.

Cette partie de l'échantillon 3, désormais appelée échantillon 3-2, et représentant l'invention, va subir un certain nombre d'expositions.

On va alors réaliser pour l'échantillon 3-2 les mesures de la concentration en ions $OH^-$ (mol/l), de la concentration en germes microbiens (nombre/gramme) et de la viscosité Brookfield™ (mPa.s).

Les résultats correspondants apparaissent dans les tableaux 9 et 10.

**Tableau 9 (échantillon 3-2)**

| | Concentration en OH⁻ (mole/l) | Concentration en germes (nombre/ml) |
|---|---|---|
| Immédiatement avant la 1ère exposition | $5 \times 10^{-5}$ | $< 10^2$ |
| Immédiatement après la 1ère exposition | $6 \times 10^{-5}$ | $2 \times 10^6$ |

Ces résultats démontrent qu'après diminution de la concentration en ions OH⁻ selon l'invention, la croissance en germes microbiens est relancée : le procédé selon l'invention permet donc de contrôler la croissance en germes microbiens dans une suspension aqueuse de matières minérales.

**Tableau 10 (échantillon 3-2)**

| | Concentration en OH⁻ (mole/l) | Viscosité Brookfield™ (mPa.s) |
|---|---|---|
| Immédiatement avant la 1ère exposition | $5 \times 10^{-5}$ | 212 |
| 1 jour après la 1ère exposition | $5 \times 10^{-5}$ | 351 |
| 5 jours après la 1ère exposition : 2ème exposition | $6 \times 10^{-5}$ | 474 |
| 12 jours après la 1ère exposition | $8 \times 10^{-5}$ | 332 |
| 26 jours après la 1ère exposition | $8 \times 10^{-5}$ | 622 |
| 26 jours après la 1ère exposition et après 5 minutes d'agitation intense | $6 \times 10^{-5}$ | 229 |

De plus, la viscosité Brookfield™ de l'échantillon selon l'invention n'est pas altérée : elle évolue de manière similaire à celle de l'échantillon non traité, représenté par l'échantillon 2.
Enfin, les viscosités Brookfield™ à 26 jours mesurées après agitation sont très proches des viscosités Brookfield™ initiales : le traitement selon l'invention permet donc de retrouver la viscosité Brookfield™.

_Echantillon 4_

Cet échantillon sert à illustrer le traitement selon l'invention, dans son mode curateur. Cet échantillon selon l'invention correspond à 1 kg de la suspension de pigments décrite au début de cet exemple et qui a été mélangée sous bonne agitation à 10 ml de suspension microbienne puis incubé dans une étuve d'incubation, pendant 7 jours à 32°C. La concentration en microbes après une semaine d'incubation était de $2 \times 10^7$ germes/ml. Cet échantillon a été mélangé sous bonne agitation, à une solution de $Ca(OH)_2$ broyé (le diamètre moyen des particules étant égal à 2 $\mu$m) contenant $2{,}6 \times 10^{-2}$ mole/l d'ions OH⁻. La viscosité Brookfield™ immédiatement après la précédente addition était de 389 mPa.s. L'échantillon 4 a été soumis à une nouvelle exposition et on a également mesuré les valeurs de la concentration en ions OH⁻ (mol/l), de la concentration en germes microbiens (nombre/gramme) et de la viscosité Brookfield™ (mPa.s).
Ces résultats apparaissent dans les tableaux 11 et 12.

**Tableau 11 (échantillon 4)**

| | Concentration en OH⁻ (mole/l) | Concentration en germes (nombre/ml) |
|---|---|---|
| 1 jour après l'exposition | $2 \times 10^{-2}$ | $< 10^2$ |
| 2 jours après l'exposition | $2 \times 10^{-2}$ | $< 10^2$ |
| 7 jours après l'exposition | $2 \times 10^{-2}$ | $< 10^2$ |

Ces résultats démontrent l'effet curateur du traitement selon l'invention.

**Tableau 12 (échantillon 4)**

| | Concentration en OH⁻ (mole/l) | Viscosité Brookfield™ (mPa.s) |
|---|---|---|
| 1 jour après l'exposition | $2 \times 10^{-2}$ | 320 |

(suite)

|  | Concentration en OH⁻ (mole/l) | Viscosité Brookfield™ (mPa.s) |
|---|---|---|
| 2 jours après l'exposition | $2 \times 10^{-2}$ | 410 |
| 7 jours après l'exposition | $2 \times 10^{-2}$ | 440 |

D'autre part, les viscosités Brookfield™ ne sont pas dégradées.

Après 7 jours, une partie de l'échantillon 4 selon l'invention ayant subi les précédentes expositions, est traitée par introduction de $CO_2$ gazeux, de manière à ramener la concentration en ions OH⁻ à une valeur égale à $3 \times 10^{-5}$ mole/l. Cette partie de l'échantillon 4, dénommé 4-2, a alors été réexposée à 1 ml de suspension microbienne. Cet instant correspond à l'instant T = 0 pour ce nouvel échantillon.

On a alors réalisé pour l'échantillon 4-2 les mesures de la concentration en ions OH⁻ (mol/l), de la concentration en germes microbiens (nombre/gramme) et de la viscosité Brookfield™ (mPa.s).

Ces résultats apparaissent dans les tableaux 13 et 14.

**Tableau 13 (échantillon 4-2)**

|  | Concentration en OH⁻ (mole/l) | Concentration en germes (nombre/ml) |
|---|---|---|
| 1 jour après l'exposition | $4 \times 10^{-5}$ | $< 10^2$ |
| 2 jours après l'exposition | $4 \times 10^{-5}$ | $< 10^2$ |
| 7 jours après l'exposition | $3 \times 10^{-5}$ | $2 \times 10^6$ |

Ces résultats démontrent que le procédé selon l'invention a permis de faire augmenter à nouveau la concentration en germes microbiens : le procédé selon l'invention permet donc de contrôler la concentration en microbes dans l'échantillon.

**Tableau 14 (échantillon 4-2)**

|  | Concentration en OH⁻ (mole/l) | Viscosité Brookfield (mPa.s) |
|---|---|---|
| 1 jour après l'exposition | $4 \times 10^{-5}$ | 360 |
| 2 jours après l'exposition | $4 \times 10^{-5}$ | 330 |
| 7 jours après l'exposition | $3 \times 10^{-5}$ | 470 |

D'autre part, les viscosités Brookfield™ ne sont pas dégradées en utilisant le procédé selon l'invention.

Une partie de l'échantillon 4, dénommée échantillon 4-3, et issue de la première conservation curatrice a été ramenée à une valeur de concentration en ions OH égale à $3 \times 10^{-5}$ mole/l par ajout de $CO_2$ liquide.

Cet échantillon a été mélangé sous bonne agitation, à une solution de $Ca(OH)_2$ broyé (le diamètre moyen des particules étant égal à 2 $\mu$m) contenant $2,6 \times 10^{-2}$ mole/l d'ions OH⁻. Cet instant est choisi comme nouvelle origine des temps.

La viscosité Brookfield™ immédiatement après la précédente addition était à présent de 425 mPa.s.

La valeur de la concentration en ions OH⁻ mesurée à 20°C était de $6,3 \times 10^{-3}$ mole/l.

On a alors déterminé pour l'échantillon 4-3 les mesures de la concentration en ions OH⁻ (mol/l), de la concentration en germes microbiens (nombre/gramme) et de la viscosité Brookfield™ (mPa.s).

Ces résultats apparaissent dans les tableaux 15 et 16.

**Tableau 15 (échantillon 4-3)**

|  | Concentration en OH⁻ (mole/l) | Concentration en germes (nombre/ml) |
|---|---|---|
| 1 jour après l'exposition | $6,3 \times 10^{-3}$ | $< 10^2$ |
| 2 jours après l'exposition | $6,3 \times 10^{-3}$ | $< 10^2$ |
| 7 jours après l'exposition | $6,3 \times 10^{-3}$ | $< 10^2$ |

**Tableau 16 (échantillon 4-3)**

|  | Concentration en OH⁻ (mole/l) | Viscosité Brookfield™ (mPa.s) |
|---|---|---|
| 1 jour après l'exposition | $6,3 \times 10^{-3}$ | 410 |
| 2 jours après l'exposition | $6,3 \times 10^{-3}$ | 440 |

Ces résultats démontrent que le procédé selon l'invention a permis de stopper la croissance microbienne par une nouvelle augmentation de la concentration en ions OH⁻ : le procédé selon l'invention permet donc de contrôler la contamination microbienne dans l'échantillon.

D'autre part, les viscosités Brookfield™ ne sont pas dégradées.

Après 7 jours, une partie de l'échantillon 4-3 selon l'invention est traitée par introduction de $CO_2$ gazeux, de manière à ramener la concentration en ions OH⁻ à une valeur égale à $3 \times 10^{-5}$ mole/l. Cette partie de l'échantillon 4, dénommée 4-4 a alors été réexposée à 1 ml de suspension microbienne.

Cet instant correspond à l'instant T = 0 pour ce nouvel échantillon.

On a alors réalisé pour l'échantillon 4-4 les mesures de la concentration en ions OH (mol/l), de la concentration en germes microbiens (nombre/gramme) et de la viscosité Brookfield™ (mPa.s).

**Tableau 17 (échantillon 4-4)**

|  | Concentration en OH⁻ (mole/l) | Concentration en germes (nombre/ml) |
|---|---|---|
| 1 jour après l'exposition | $2,5 \times 10^{-5}$ | $< 10^2$ |
| 2 jours après l'exposition | $2,5 \times 10^{-5}$ | $2 \times 10^6$ |

Ces résultats démontrent que le procédé selon l'invention a permis de faire augmenter à nouveau la concentration en germes microbiens : le procédé selon l'invention permet donc de contrôler la concentration en microbes dans l'échantillon.

**Tableau 18 (échantillon 4-4)**

|  | Concentration en OH⁻ (mole/l) | Viscosité Brookfield™ (mPa.s) |
|---|---|---|
| 1 jour après l'exposition | $2,5 \times 10^{-5}$ | 560 |
| 2 jours après l'exposition | $2,5 \times 10^{-5}$ | 530 |
| 7 jours après l'exposition | $2,5 \times 10^{-5}$ | 570 |

D'autre part, les viscosités Brookfield™ ne sont pas dégradées en utilisant le procédé selon l'invention.

**Exemple 3**

Cet exemple a pour but d'illustrer le procédé selon l'invention, dans son mode curateur, appliqué à une suspension aqueuse de matière minérale qui est du kaolin.

Il a également pour but d'illustrer que le procédé selon l'invention permet de contrôler l'évolution de la croissance de germes microbiens dans une telle suspension.

*Suspension de pigments*

On a fabriqué une suspension aqueuse contenant 63,3 % en poids sec de kaolin américain (Géorgie) (dont 95 % en poids des particules ont un diamètre inférieur à 2 $\mu$m, et 70 % en poids des particules ont un diamètre inférieur à 1 $\mu$m), par broyage à une concentration de 25 % en poids puis séchage dans un sécheur à pulvérisation et dispersion dans de l'eau avec utilisation de 0,25 % en poids sec par rapport au poids sec de matières minérales de polyacrylate de sodium du commerce.

La valeur de pH de la suspension après broyage était de 7,7 mesurée à 20°C.

On a préparé 2 échantillons de 1 kg à partir de la suspension de pigment.

*Suspension microbienne*

On a préparé un mélange de 7 types de bactéries différents, gramme-négatives, principalement formé à partir de la famille des pseudo-monades (majoritairement, Pseudomonas aeruginosa) isolés à partir de suspension de carbonate de calcium ayant subie une germination naturelle, provenant d'Autriche.

Les 7 différentes variétés de microbes ont pu être identifiées grâce au test API™ bien connu de l'homme du métier, et mis au point par la société BIOMERIEUX™.

La concentration en microbes est de $5 \times 10^6$ germes/ml.

*Echantillon 5*

L'échantillon 5 correspondant à 1 kg de ladite suspension de pigments a été mélangé sous bonne agitation à 0,053 mole de d'ions OH⁻ (ajouté sous la forme d'une solution de CaO dans l'éthylène glycol à une concentration de 2,7 M).

La viscosité Brookfield™ de la suspension aqueuse de kaolin immédiatement après addition du CaO était de 327 mPa.s.

La valeur de la concentration en ions OH⁻ était de $1,25 \times 10^{-2}$ mole/l.

*Echantillon 6*

L'échantillon 6 illustre l'art antérieur et correspond au mélange de la suspension de pigment et de la suspension microbienne.

En utilisant les notations précédentes, les échantillons 6 et 7 ont subi un certain nombre d'expositions et on a mesuré alors les valeurs de la concentration en ions OH⁻ (mol/l), de la concentration en germes microbiens (nombre/gramme) et de la viscosité Brookfield™ (mPa.s).

Ces résultats apparaissent dans les tableaux 19 et 20.

**Tableau 19 (échantillons 5 et 6)**

| | Concentration en OH⁻ (mole/l) | | Concentration en germes (nombre/ml) | |
|---|---|---|---|---|
| | Echantillon 5 | Echantillon 6 | Echantillon 5 | Echantillon 6 |
| Immédiatement avant la 1ère exposition | $1,25 \times 10^2$ | $5 \times 10^{-7}$ | $<10^2$ | $<10^2$ |
| 1ère exposition | $1,25 \times 10^{-2}$ | $5 \times 10^{-7}$ | $<10^2$ | $4 \times 10^5$ |
| 3 jours après 1a 1ère exposition : 2ème exposition | $1,25 \times 10^{-2}$ | $5 \times 10^{-7}$ | $<10^2$ | $9 \times 10^6$ |
| 4 jours après la 2ème exposition | $1,25 \times 10^{-2}$ | $5 \times 10^{-7}$ | $<10^2$ | $6 \times 10^7$ |

Ces résultats illustrent l'efficacité du procédé selon l'invention dans son aspect protecteur.

**Tableau 20 (échantillons 5 et 6)**

| | Concentration en OH⁻ (mole/l) | | Viscosité Brookfied (mPa.s) | |
|---|---|---|---|---|
| | Echantillon 5 | Echantillon 6 | Echantillon 5 | Echantillon 6 |
| Immédiatement avant la 1ère exposition | $1,25 \times 10^{-2}$ | $5 \times 10^{-7}$ | 444 | 394 |
| 1 jour après la 1ère exposition | $1,25 \times 10^{-2}$ | $5 \times 10^{-7}$ | 518 | 463 |
| 5 jours après la 1ère exposition : 2ème exposition | $1,25 \times 10^{-2}$ | $5 \times 10^{-7}$ | 804 | 676 |
| 4 jours après la 2ème exposition | $1,25 \times 10^{-2}$ | $5 \times 10^{-7}$ | 1150 | 855 |
| 26 jours après la 1ère exposition | $1,25 \times 10^{-2}$ | $5 \times 10^{-7}$ | 1907 | 1190 |
| 26 jours après la 1ème exposition et après 5 minutes d'agitation intense | $1,25 \times 10^{-2}$ | $5 \times 10^{-7}$ | 565 | 444 |

D'autre part, les viscosités Brookfield™ de l'échantillon selon l'invention ne sont pas dégradées par rapport à l'échantillon représentant l'art antérieur.

Après 26 jours, une partie de l'échantillon 5 selon l'invention ayant subi les précédentes expositions, est traitée

par introduction de $CO_2$ gazeux, de manière à ramener la concentration en ions OH- à une valeur de $2 \times 10^{-6}$ mole/l.
Cet instant correspond à l'instant T = 0 pour ce nouvel échantillon.
Cette partie de l'échantillon 5, désormais appelée échantillon 5-2, et représentant l'invention, va subir un certain nombre d'expositions.

On va alors réaliser pour l'échantillon 5-2 les mesures de la concentration en ions OH- (mol/l), de la concentration en germes microbiens (nombre/gramme) et de la viscosité Brookfield™ (mPa.s).
Ces résultats apparaissent dans les tableaux 21 et 22.

**Tableau 21 (échantillon 5-2)**

|  | Concentration en OH- (mole/l) | Concentration en germes (nombre/ml) |
|---|---|---|
| Immédiatement avant la 1ère exposition | $2 \times 10^{-6}$ | $< 10^2$ |
| Exposition | $4 \times 10^{-6}$ | $2 \times 10^6$ |

Ces résultats démontrent que le procédé selon l'invention a permis de faire augmenter à nouveau la concentration en germes microbiens : le procédé selon l'invention permet donc de contrôler la croissance microbienne dans la suspension aqueuse de matière minérale.

**Tableau 22 (échantillon 5-2)**

|  | Concentration en OH- (mole/l) | Viscosité Brookfield™ (mPa.s) |
|---|---|---|
| Immédiatement avant la 1ère exposition | $2 \times 10^{-6}$ | 638 |
| 1 jour après la 1ère exposition | $2 \times 10^{-6}$ | 680 |
| 5 jours après la 1ère exposition : 2ème exposition | $3 \times 10^{-6}$ | 790 |
| 12 jours après la 1ère exposition | $4 \times 10^{-6}$ | 948 |
| 26 jours après la 2ème exposition | $8 \times 10^{-6}$ | 1198 |
| 26 jours après la 1ère exposition et après 5 minutes d'agitation intense | $6 \times 10^{-6}$ | 688 |

D'autre part, les viscosités Brookfield™ ne sont pas dégradées par rapport à l'échantillon représentant l'art antérieur.

**Exemple 4**

Cet exemple a pour but d'illustrer le procédé selon l'invention, dans ses modes curateur et protecteur, appliqué à une suspension aqueuse de matière minérale qui est du kaolin, et dans le cas où le procédé selon l'invention met en oeuvre un biocide selon l'étape d) qui est du o-phénylphénol.
Il a également pour but d'illustrer que le procédé selon l'invention permet de contrôler l'évolution de la croissance de germes microbiens dans une telle suspension.

Suspension de pigments

On a préparé une suspension aqueuse contenant 78,3 % en poids sec de carbonate de calcium naturel qui est du marbre (dont 90 % en poids des particules ont un diamètre inférieur à 2 $\mu$m, et 65 % en poids des particules ont un diamètre inférieur à 1 $\mu$m), obtenu par broyage avec utilisation de 0,65 % en poids sec par rapport au poids sec de matières minérales d'un polyacrylate du commerce neutralisé par un mélange sodium/magnésium.
La valeur de pH de la suspension après le broyage était de 9,7, mesurée à 20°C.
2 échantillons de 1 kg ont été préparés à partir de la suspension de pigment.

*Suspension microbienne*

On a préparé un mélange, formé de 7 types de bactéries gramme-négatives différentes principalement composé depuis la famille des pseudo-monades (majoritairement Pseudomonas aeruginosa), isolé à partir de suspensions de carbonate de calcium ayant germées de façon naturelle, provenant d'Autriche.

Les 7 différentes variétés de microbes ont pu être identifiées grâce au test API™ bien connu de l'homme du métier, et mis au point par la société BIOMERIEUX™.

La concentration en microbes est de 5 x 10$^6$ germes/ml.

*Echantillon 7*

Cet échantillon sert à illustrer le procédé selon l'invention dans son mode curateur, et en combinaison avec un biocide qui est du o-phénylphénol.

Cet échantillon selon l'invention correspondant à 1 kg de ladite suspension de pigments a été mélangé sous agitation à 10 ml de suspension microbienne et ensuite incubé pendant 7 jours à 32°C dans une étuve d'incubation.

La concentration en bactéries au bout d'une semaine d'incubation était de 2 x 10$^7$ germes /ml.

Ensuite, on a ajouté à l'échantillon 200 ppm de o-phénylphénol sous bonne agitation, sous forme d'une solution de o-phénylphénol à 45 %, dissout dans une solution contenant du KOH à raison de 1,07 moles KOH par mole de o-phénylphénol. On a également ajouté 1270 ppm de $Ca(OH)_2$, à titre de suspension finement broyée (le diamètre moyen des particules après broyage étant de 2 $\mu$m) à une concentration de 2,7 molaire en $Ca(OH)_2$. La viscosité Brookfield™ de la suspension de carbonate de calcium immédiatement après addition du $Ca(OH)_2$ était de 271 mPa.s.

Cet échantillon selon l'invention a été ensuite plusieurs fois mélangé à 10 ml de suspension microbienne puis chaque fois incubé dans une étuve d'incubation, pendant 24 heures à 30°C.

On a ensuite déterminé les valeurs de la concentration en ions OH$^-$ (mol/l), de la concentration en germes microbiens (nombre/gramme) et de la viscosité Brookfield™ (mPa.s).

Ces résultats apparaissent dans les tableaux 23 et 24.

**Tableau 23 (échantillon 7)**

| | Concentration en OH$^-$ (mole/l) | Concentration en germes (nombre/ml) |
|---|---|---|
| 1 jour après l'exposition | 1,58 x 10$^{-2}$ | < 10$^2$ |
| 2 jours après J'exposition | 1,58 x 10$^{-2}$ | < 10$^2$ |
| 7 jours après l'exposition | 1,58 x 10$^{-2}$ | < 10$^2$ |

Ces résultats démontrent que le procédé selon l'invention permet d'obtenir un traitement curateur de la suspension aqueuse de matière minérale.

**Tableau 24 (échantillon 7)**

| | Concentration en OH$^-$ (mole/l) | Viscosité Brookfield™ (mPa.s) |
|---|---|---|
| Immédiatement avant exposition | 1,58 x 10$^{-2}$ | 271 |
| 1 jour après exposition | 1,58 x 10$^{-2}$ | 698 |
| 4 jours après exposition | 1,58 x 10$^{-2}$ | 823 |
| 25 jours après exposition | 1,58 x 10$^{-2}$ | 872 |
| 25 jours après exposition et après 5 minutes d'agitation intense | 1,58 x 10$^{-2}$ | 282 |

D'autre part, les viscosités Brookfield™ de la suspension aqueuse de matière minérale selon l'invention ne sont pas dégradées.

*Echantillon 8*

Cet échantillon sert à illustrer l'art antérieur et met en oeuvre un biocide qui est l'o-phénylphénol.

Le deuxième échantillon selon l'invention correspondant à 1 kg de ladite suspension de pigments a été mélangé sous bonne agitation avec 10 ml de suspension microbienne puis incubé dans une étuve d'incubation, pendant 7 jours à 32°C.

La concentration microbienne au bout d'une semaine d'incubation était de 2 x 10$^7$ germes/ml.

Ensuite, on a ajouté à ce deuxième échantillon sous bonne agitation 200 ppm de o-phénylphénol, sous forme d'une solution de o-phénylphénol à 45 %, dissous par 1,07 mole de KOH par mole de o-phénylphénol.

La viscosité Brookfield™ immédiatement après l'addition d'o-phénylphénol était de 285 mPa.s.

On a ensuite déterminé les valeurs de la concentration en ions OH$^-$ (mol/l), et de la concentration en germes microbiens (nombre/gramme).

Ces résultats apparaissent dans le tableau 27.

**Tableau 27 (échantillon 8)**

|  | Concentration en OH$^-$ (mole/l) | Concentration en germes (nombre/ml) |
|---|---|---|
| 1 jour après l'exposition | $6,3 \times 10^{-5}$ | $> 10^2$ |
| 2 jours après l'exposition | $6,3 \times 10^{-5}$ | $> 10^4$ |
| 7 jours après l'exposition | $6,3 \times 10^{-5}$ | $> 10^5$ |

L'échantillon 8, représentant l'art antérieur, montre que l'o-phénylphénol présente un effet microbicide insuffisant, sur les germes utilisés dans la plage curatrice.

La conservation est incomplète et n'est pas suffisante. A l'inverse, avec l'échantillon 7 selon l'invention, on a pu montrer que le procédé selon l'invention qui combine l'emploi de l'o-phénylphénol et une étape d'augmentation de la concentration en ions OH$^-$, permet d'obtenir de très bons résultats au niveau de la diminution des germes microbiens dans la suspension aqueuse de matière minérale.

**Exemple 5**

Cet exemple a pour but d'illustrer le procédé selon l'invention dans ses modes curateur et protecteur sur des eaux de lavage de wagons.

*Conservation d'eaux de lavage de wagon*

Afin de simuler des eaux de lavage de wagon, on a utilisé une solution de sel de cuisine tamponnée, comportant 3 % en poids d'une suspension de carbonate de calcium, issu de l'exemple 1.

L'essai de désinfection a été effectué sur deux échantillons différents.

*Echantillon 9*

Cet échantillon sert à illustrer le procédé selon l'invention dans son mode protecteur.

Le Ca(OH)$_2$ a été dissout dans une solution tamponnée de phosphate stérile à 0,85 % en poids (PBS) et a été mélangé après 24 heures de stockage à 30°C avec le cocktail bactéries/levures indiqué dans le tableau 28.

Les échantillons ont été encore incubés pendant 24 heures à 30°C et ensuite sortis des plaques.

Les germes ayant crû, ont été examinés au microscope optique.

*Echantillon 10*

Cet échantillon sert à illustrer le procédé selon l'invention dans son mode curateur.

Un cocktail de bactéries (dans du PBS), d'une part, et un cocktail bactéries/levures dans du (PBS), d'autre part, ont été mélangés avec du Ca(OH)$_2$, stockés puis démoulés.

**Tableau 28 (Composition du cocktail de microbes)**

| Type de bactéries | Remarques |
|---|---|
| **Cocktail de bactéries sans levure :** | |
| Pseudomonas aeruginosa | Gramme-négatif, non Enterobacteriacae |
| Pseudomonas Pseudoalcaligenes | Gramme-négatif, non Enterobacteriacae |
| Pseudomonas stuzeri | Gramme-négatif, non Enterobacteriacae |
| Acinetobacter baumannii/calco | Gramme-négatif, enterobacteriacae |
| Klebsiella spp. | Gramme-négatif, enterobacteriacae |
| Bazillus subtilis | Gramme-positif, formateur de sports |

(suite)

| Type de bactéries | Remarques |
|---|---|
| **Cocktail de bactéries sans levure :** | |
| Bazillus spp. | Gramme-positif, |
| Staphylococcus cohnii cohnii | Gramme-positif, |
| Koc. Varians/rosea | Gramme-positif, |
| Micrococcus kristae | Gramme-positif, |
| | |
| **« Levures » et autres :** | |
| Candida albicans, eucaryote, monocellulaire fortement développeur | Levure |
| Germe Rosa isolé depuis une solution de polyacrylate de sodium, résistant au formaldéhyde | non spécifié plus en détail |
| Germe Rosarot isolé à partir d'un circuit de machine à papier, germe à valeur gramme instable | non spécifié en détail |

Les tableaux 29 et 30 indiquent la concentration en ions OH⁻ (mole/l) et la concentration en germes et levures (nombre/ml) mesurée dans les échantillons, après 24 heures d'incubation et ce, pour différentes concentration initiales de Ca(OH)$_2$.

**Tableau 29 (échantillon 9)**

| | 0 ppm | 200 ppm [1] | 500 ppm [1] | 1000 ppm [1] | 2000 ppm [1] |
|---|---|---|---|---|---|
| concentration en germes microbiens (nombre/ml) | $10^3$ | $10^3$ | 30 | <10 | <10 |
| concentration en ions OH⁻ (mole/l) | $3 \times 10^{-7}$ | $5 \times 10^{-4}$ | $3 \times 10^{-3}$ | $1 \times 10^{-2}$ | $5 \times 10^{-2}$ |
| *1) Dosage du Ca(OH)$_2$ : Quantité active/Globale* | | | | | |

**Tableau 30 (échantillon 10)**

| | 0ppm | 200ppm [1] | 500ppm [1] | 1000ppm [1] |
|---|---|---|---|---|
| germes microbiens (nombre/ml) | $10^5$ | $10^5$ | 30 | < 10 |
| concentration en ions OH⁻ (mole/l) | $3 \times 10^{-7}$ | $4 \times 10^{-5}$ | $3 \times 10^{-3}$ | $1 \times 10^{-2}$ |
| *1) Dosage du Ca(OH)$_2$ Quantité active/Globale* | | | | |

Ainsi, le tableau 29 démontre que pour une concentration initiale en Ca(OH)$_2$ de 200 ppm, on obtient après 24 heures une concentration en germes microbiens de 1000/ml, et une concentration en ions OH⁻ de $5 \times 10^{-4}$ mole/l.

La valeur de la concentration en germes microbiens a été réduite à 50/ml, pour une concentration initiale en Ca(OH)$_2$ de 500 ppm ; on obtient alors une concentration en ions OH⁻ de $3 \times 10^{-3}$ mole/l.

Ces résultats démontrent bien l'effet protecteur du procédé selon l'invention.

Enfin, la valeur de la concentration en germes microbiens est réduite à une quantité inférieure à 10 par ml pour une concentration initiale en Ca(OH)$_2$ de 500 ppm ; on obtient alors une concentration en ions OH⁻ de $3 \times 10^{-3}$ mole/l.

Ce dernier résultat illustre le fait qu'avec le procédé selon l'invention utilisé dans son mode protecteur, on peut obtenir des suspensions aqueuses de matières minérales comportant une très faible quantité de germes microbiens, notamment une quantité inférieure à 10 par ml.

Parallèlement, le tableau 30 démontre l'efficacité du procédé selon l'invention dans son mode curateur puisqu'au bout de 24 heures, la concentration en microbes diminue d'autant plus que la concentration initiale en Ca(OH)$_2$ est importante.

On note ainsi qu'avec une concentration initiale en Ca(OH)$_2$ de 1000 ppm, une très large partie des germes microbiens est détruite puisque leur concentration est inférieure à 10 par ml au bout de 24 heures.

Ce dernier résultat illustre le fait qu'avec le procédé selon l'invention utilisé dans son mode curateur, on peut obtenir des suspensions aqueuses de matières minérales comportant une très faible quantité de germes microbiens, notamment une quantité inférieure à 10 par ml.

Les échantillons 9 et 10 de concentration initiale en $Ca(OH)_2$ égale à 1000 ppm ont ensuite été démoulés à 30°C sur un milieu bactérien dit PCA (Plate Count Agar).

On indique que ces milieux correspondent à une formulation bactérienne décrite dans les ouvrages : « American Public Health Association : Standard Methods for the Examination of Dairy Products, 15th ed., 1985 », « American Public Health Association, American Water Works Association and Water Pollution Control Federation : Standard Methods for the Examination of Water and Wastewater, 20th ed., Washington, 1998 » et « An improved agar medium for the detection of proteolytic organisms in total bacterial counts, J. Appl. Bact., 33; 363-370 (1970) ».

Après 48 heures d'incubation, les échantillons sont à nouveau démoulés sur le même milieu de type PCA.

On dénombre alors au niveau des germes microbiens moins de 40 bactéries par ml et moins de 100 microbes autres que des bactéries par ml.

)Ces résultats illustrent donc l'efficacité du procédé selon l'invention dans son mode curateur.

Pour une partie de l'échantillon 9, dénommée 9-2, la concentration en ions OH⁻ a été ramenée à une valeur de $5 \times 10^{-7}$ mole/l par ajout de $CO_2$ gazeux. Elle a été de nouveau incubée pendant 24 heures en présence du cocktail de bactéries et de levures décrit dans le tableau 28. Elle a ensuite été démoulée sur un milieu bactérien de type PCA tel que décrit auparavant.

Comme précédemment, on a alors mesuré la concentration en germes microbiens en fonction de la concentration initiale en $Ca(OH)_2$ : ces résultats figurent dans le tableau 31.

### Tableau 31 (échantillon 9-2)

| | 0 ppm | 200 ppm [1] | 1000 ppm [1] |
|---|---|---|---|
| concentration en germes microbiens (nombre/ml) | >10⁴ | >10⁵ | >10⁵ |
| concentration en ions OH⁻ (mole/l) | $1 \times 10^{-6}$ | $1 \times 10^{-6}$ | $1 \times 10^{-6}$ |
| [1] Dosage du $Ca(OH)_2$ : Quantité active/Globale | | | |

Ces résultats démontrent que par diminution de la concentration en ions OH⁻ grâce à l'ajout de $CO_2$ liquide on a pu, grâce au procédé selon l'invention, supprimer l'effet protecteur et relancer la croissance microbienne.

### Exemple 6

Cet exemple illustre le procédé selon l'invention dans lequel l'étape de diminution de la concentration en ions OH⁻ est combinée à un procédé physique qui est ici un procédé basé sur l'augmentation de la température.

### Suspension de pigments

On a préparé une suspension aqueuse contenant 0,1 % en poids sec de carbonate de calcium naturel qui est du marbre (dont 90 % en poids des particules ont un diamètre inférieur à 2 μm, et 65 % en poids des particules ont un diamètre inférieur à 1 μm), obtenu par broyage avec utilisation de 0,65 % en poids sec par rapport au poids sec de matières minérales d'un polyacrylate du commerce neutralisé par un mélange sodium/magnésium.
On a préparé à chaque fois 2 échantillons.

### Suspension de microbes

On a préparé une suspension de microbes de concentration égale à 10⁴ microbes/g, et dont la composition est donnée par le tableau 32.

### Echantillon 11

Cet échantillon illustre l'invention et consiste en la suspension de pigments à laquelle on a mélangé la suspension de microbes, et dans laquelle on a introduit une solution contenant 500 ppm de $Ca(OH)_2$.
Cet échantillon a été incubé pendant 24 heures à 20°C.

*Echantillon 12*

Cet échantillon illustre l'art antérieur et est identique à l'échantillon 11 sauf qu'il ne contient pas de $Ca(OH)_2$. Cet échantillon a été incubé pendant 24 heures à 20°C.

*Echantillon 13*

Cet échantillon illustre l'invention et consiste en la suspension de pigments à laquelle on a mélangé la suspension de microbes, et dans laquelle on a introduit une solution contenant 500 ppm de $Ca(OH)_2$. Cet échantillon a été incubé pendant 24 heures à 40°C.

*Echantillon 14*

Cet échantillon illustre l'art antérieur et est identique à l'échantillon 13 sauf qu'il ne contient pas de $Ca(OH)_2$. Cet échantillon a été incubé pendant 24 heures à 40°C.

Tableau 32 (composition du cocktail de microbes)

| Type de bactéries | Remarques |
|---|---|
| **Cocktail de bactéries sans levure :** | |
| Pseudomonas aeruginosa | Gramme-négatif, non Enterobacteriacae |
| Pseudomonas Pseudoalcaligenes | Gramme-négatif, non Enterobacteriacae |
| Pseudomonas stuzeri | Gramme-négatif, non Enterobacteriacae |
| Acinetobacter baumannii/calco | Gramme-négatif, enterobacteriacae |
| Klebsiella spp. | Gramme-négatif, enterobacteriacae |
| Bazillus subtilis | Gramme-positif, formateur de sports |
| Bazillus spp. | Gramme-positif, |
| Staphylococcus cohnii | Gramme-positif, |
| Koc. Varians/rosea | Gramme-positif, |
| Micrococcus kristae | Gramme-positif, |
| | |
| **« Levures » et autres :** | |
| Candida albicans, eucaryote, monocellulaire fortement développeur | Levure |
| Germe Rosa isolé depuis une solution de polyacrylate de sodium, résistant au formaldéhyde | non spécifié plus en détail |
| Germe Rosarot isolé à partir d'un circuit de machine à papier, germe à valeur gramme instable | non spécifié en détail |

On a alors mesuré pour chaque échantillon la concentration en ions $OH^-$ (mole/l) ainsi que la concentration en bactéries et la concentration en microbes autres que des bactéries (nombre/ml) ; ces résultats apparaissent dans le tableau 33.

**Tableau 33**

| | Echantillon 12 | Echantillon 14 | Echantillon 11 | Echantillon 13 |
|---|---|---|---|---|
| Température | 20°C | 40°C | 20°C | 40°C |
| Concentration initiale en $Ca(OH)_2$ (ppm) | 0 | 500 | 0 | 500 |
| Concentration en bactéries (nombre/ml) | > $10^4$ | > $10^4$ | 2 x $10^4$ | <10 |

(suite)

| | Echantillon 12 | Echantillon 14 | Echantillon 11 | Echantillon 13 |
|---|---|---|---|---|
| Concentration en microbes autres que bactéries (nombre/ml) | > $10^4$ | > $10^4$ | $2 \times 10^3$ | <10 |
| Concentration en ions OH⁻ (mole/l) | $2 \times 10^{-7}$ | $2 \times 10^{-7}$ | $3 \times 10^{-3}$ | $3 \times 10^{-3}$ |

Le tableau 33 démontre que l'étape de réduction de la concentration en ions OH⁻, représentative du procédé selon l'invention, permet à 20°C de réduire le nombre de germes microbiens de tous genres (échantillon 11).

Il démontre également que cette étape de réduction de la concentration en ions OH⁻, en combinaison avec un procédé physique qui est l'augmentation de la température à 40°C, ladite combinaison étant aussi représentative du procédé selon l'invention, permet de réduire de façon très marquée la concentration en microbes de tous genres, puisque celle-ci est dès lors inférieure à 10 par ml.

Pour les échantillons 11 et 13 selon l'invention, on a ensuite ramené la concentration en ions OH⁻ à une valeur de $3,2 \times 10^{-6}$ mole/l par ajout de $CO_2$. Ces échantillons ont par la suite été à nouveau incubés dans le cocktail microbien décrit précédemment puis démoulés sur un cocktail microbien de type PCA à 30°C, et on a laissé ledit cocktail agir pendant 24 heures.

La concentration en ions OH⁻ est alors égale à $1 \times 10^{-4}$ mole/l et on observe une nouvelle augmentation des germes microbiens : au moyen du procédé selon l'invention, on a donc pu relancer la croissance microbienne.

**Exemple 7**

Cet exemple a pour but d'illustrer le procédé selon l'invention, dans son mode curateur en broyage, appliqué à une suspension aqueuse de matière minérale qui est du carbonate de calcium.
Il a également pour but d'illustrer que le procédé selon l'invention permet de contrôler l'évolution de la croissance de germes microbiens dans une telle suspension, sans altérer sa stabilité en terme de viscosité Brookfield™.

*Suspension microbienne*

On a préparé un mélange de 7 types de bactéries gramme-négatives différentes, principalement composé d'après la famille des pseudo-monades (majoritairement du pseudomonas aeruginosa), isolé à partir d'une boue de carbonate de calcium ensemencée naturellement par des germes, provenant d'Autriche.

Les 7 différentes variétés de microbes ont pu être identifiées grâce au test API™ bien connu de l'homme du métier, et mis au point par la société BIOMERIEUX™.

La concentration en microbes est de $5 \times 10^6$ germes/ml.

*Echantillon 15*

Cet échantillon sert à illustrer l'art intérieur ; il est obtenu par exposition d'une suspension de carbonate de calcium à la suspension microbienne, puis par broyage de ladite suspension de carbonate de calcium.

On a préparé 5 kg en carbonate de calcium sec, à partir d'une suspension aqueuse à 77,3 % en poids sec de marbre naturel (dont 30 % en poids des particules ont un diamètre inférieur à 2 $\mu$m, et 8 % en poids des particules ont un diamètre inférieur à 1 $\mu$m).

Ladite suspension a été traitée avec 10 ml de la suspension microbienne et stockée pendant 24 heures à 30°C.

Par broyage avec 0,65% en poids sec par rapport au poids sec de matières minérales d'un polyacrylate neutralisé par un mélange de sodium et de magnésium du commerce, on a fabriqué une suspension dont 88 % en poids des particules ont un diamètre inférieur à 2 $\mu$m, et 64 % en poids des particules ont un diamètre inférieur à 1 $\mu$m.

La valeur de pH de la suspension après le broyage était de 9,7 mesurée à 20°C.

La viscosité Brookfield™ 24 heures après broyage était de 284 mPa.s.
La concentration en germes microbiens de cette suspension après broyage était supérieure à $10^5$ germes/ml.

*Echantillon 16*

Cet échantillon sert à illustrer le traitement selon l'invention, dans son mode protecteur. On a préparé 5 kg en carbonate de calcium sec, à partir d'une suspension aqueuse à 77,1 % en poids de marbre naturel (dont 30 % en poids des particules ont un diamètre inférieur à 2 $\mu$m, et 8 % en poids des particules ont un diamètre inférieur à 1 $\mu$m).

Ladite suspension a été traitée avec 10 ml de la suspension microbienne et stockée pendant 24 heures a 30°C.

Par la suite, cet échantillon a été mélangé sous bonne agitation, à une solution de $Ca(OH)_2$ broyé (le diamètre moyen des particules étant égal à 2 $\mu$m) contenant 2 x $10^{-2}$ mole/l d'ions $OH^-$.

Par broyage avec 0,65% en poids sec par rapport au poids sec de matières minérales d'un polyacrylate neutralisé par un mélange de sodium et de magnésium du commerce on a fabriqué une suspension dont 91 % en poids des particules ont un diamètre inférieur à 2 $\mu$m, et 66 % en poids des particules ont un diamètre inférieur à 1 $\mu$m.

La valeur du pH de la suspension après le broyage était de 12,2 mesurée à 20°C.

La viscosité Brookfield™ mesurée 24 heures après la précédente addition était de 253 mPa.s.
La concentration en germes de cette suspension après broyage était inférieure à $10^2$ germes/ml.

Ces résultats démontrent que le procédé selon l'invention a permis de réduire très nettement la contamination microbienne de l'échantillon par rapport au traitement de l'art antérieur, sans pour autant altérer la stabilité de l'échantillon selon l'invention en terme de viscosité Brookfield™.

Après 2 jours, l'échantillon 16 selon l'invention a été traité par introduction de $CO_2$ gazeux, de manière à ramener la concentration en ions $OH^-$ à une valeur égale à 2 x $10^{-5}$ mole/l.

La viscosité Brookfield™ 24 heures après la précédente addition de $CO_2$ était de 222 mPa.s.

La concentration en germes de cette suspension après broyage était inférieure à $10^2$ germes/ml.

Par la suite, l'échantillon 16 selon l'invention a été traité de nouveau par introduction de $CO_2$ gazeux.

Il a ensuite été traité avec 1 ml de suspension microbienne et stocké à 30 °C pendant 48 heures. La concentration en germes de cette suspension était alors supérieure à $10^6$ germes/ml. Ces résultats démontrent que le procédé selon l'invention, par addition de $CO_2$ gazeux, a permis de relancer la croissance microbienne dans l'échantillon.

**Exemple 8**

Cet exemple a pour but d'illustrer le procédé selon l'invention, dans son mode curateur, appliqué à une suspension aqueuse de matière minérale qui est un carbonate de calcium précipité.
Il a également pour but d'illustrer que le procédé selon l'invention permet de contrôler l'évolution de la croissance de germes microbiens dans une telle suspension.

*Suspension de pigments* :

On a fabriqué une suspension aqueuse contenant 50,0 % en poids sec de de carbonate precipite de type Syncarb™ F 474 commercialisé par la société OMYA™. La valeur du pH de la suspension était de 9,7, mesurée à 20°C.
On a préparé 2 échantillons de 1 kg à partir de la suspension de pigment.

*Suspension bactérienne :*

On a préparé un mélange de 7 types de bactéries différents, gramme-négatives, principalement formé à partir de la famille des pseudo-monades (majoritairement, Pseudomonas aeruginosa) isolées à partir d'une suspension de carbonate de calcium ayant subi une germination naturelle, provenant d'Autriche.

La concentration en germes est de 2 x $10^5$ germes/ml.

*Echantillon 17*

Le premier échantillon de 1 kg de suspension de carbonate de calcium précipité, a été mélangé sous bonne agitation à 0,075 mole de d'ions $OH^-$ (ajouté sous la forme d'une suspension à 2.7 molaire de $Ca(OH)_2$ dans l'eau).
La viscosité de la suspension de carbonate de calcium précipité immédiatement après l'addition de $Ca(OH)_2$ était égale 227 mPa.s.
Le pH de la suspension était égal à 12.1.
Cette suspension correspond désormais à l'échantillon 17 qui illustre l'invention.

*Echantillon 18*

L'autre échantillon de 1 kg de suspension de carbonate de calcium précipité a été mélangé à la suspension bactérienne.
La viscosité de cette suspension était égale à 257 mPa.s.
Cette suspension correspond désormais à l'échantillon 18 qui illustre l'art antérieur.

Les échantillons 17 et 18 ont ensuite subi un certain nombre d'expositions, tel qu'indiqué dans le tableau 34. On a alors déterminé pour chacun d'eux la concentration en ions $OH^-$ ainsi que la concentration en germes microbiens à différents instants (selon les méthodes précédemment décrites), tel qu'indiqué également dans le tableau 34.

**Tableau 34**

| Temps (T = nombre de jours) | Evénement (Exposition et/ou mesures) | Concentration en OH-(mole/l)) | | Concentration en germes (nombre/ml) | |
|---|---|---|---|---|---|
| | | **Echantillon 17** | **Echantillon 18** | **Echantillon 17** | **Echantillon 18** |
| T=0 | Mesures | $2x\ 10^{-2}$ | $7,5\ x\ 10^{-5}$ | $<10^2$ | $3.3\ 10^4$ |
| T = 2 | Exposition et mesures | $2\ x\ 10^{-2}$ | $1\ x\ 10^{-4}$ | $<10^2$ | $>10^5$ |
| T=4 | Exposition et mesures | $2\ x\ 10^{-2}$ | $1\ x\ 10^{-4}$ | $<10^2$ | $\gg 10^5$ |

Ces résultats illustrent l'efficacité du procédé selon l'invention dans son aspect protecteur. D'autre part, les viscosités de l'échantillon n° 17 selon l'invention mesurées à T=2 jours et T=4 jours sont égales à 227 mPa.s et 232 mPa.s : elles ne sont donc pas dégradées par rapport à l'échantillon représentant l'art antérieur.

Après 4 jours, une partie de l'échantillon 17 selon l'invention est traitée par introduction de $CO_2$ gazeux, de manière à ramener la concentration en ions OH- à une valeur de $4\ x\ 10^{-6}$ mole/litre.

Cette partie de l'échantillon 17 est désormais appelée échantillon 17 bis.

Cet échantillon 17 bis a ensuite subi un certain nombre d'expositions, tel qu'indiqué dans le tableau 35.

On a alors déterminé la concentration en ions OH- ainsi que la concentration en germes microbiens à différents instants (selon les méthodes précédemment décrites), tel qu'indiqué dans le tableau 35 (l'instant T=0 correspond au moment de l'introduction de $CO_2$ gazeux dans l'échantillon 17 dès lors appelé échantillon 17 bis).

**Tableau 35**

| Temps (T = nombre de jours) | Evénement (exposition et/ou mesures) | Concentration en OH- (mole/litre) | Concentration en germes (nombre/ml) |
|---|---|---|---|
| T = 0 | Mesures | $4\ x\ 10^{-6}$ | $<10^2$ |
| T = 2 jours | Exposition Mesures | $4\ x\ 10^{-6}$ | $2.7\ 10^3$ |
| T = 4 jours | Exposition Mesures | $4\ x\ 10^{-6}$ | $>10^5$ |

Ces résultats démontrent que le procédé selon l'invention a permis de faire augmenter à nouveau la concentration en germes : le procédé selon l'invention permet donc de contrôler la croissance microbienne dans la suspension aqueuse de carbonate de calcium précipité.

**Revendications**

1.  Procédé de désinfection et/ou de conservation et/ou de réduction et/ou de contrôle de la contamination microbienne de dispersions aqueuses et/ou de suspensions aqueuses de matières minérales choisies parmi les carbonates des calcium naturels et/ou précipités et leurs mélanges, **caractérisé en ce qu'**il met en oeuvre ;

    a) au moins une étape d'augmentation de la concentration en ions OH- desdites dispersions et/ou desdites suspensions aqueuses, à une valeur supérieure ou égale à $1\ x\ 10^{-2}$ mole/l,
    b) au moins une étape de dispersion et/ou de broyage desdites dispersions et/ou suspensions aqueuses, intervenant avant, pendant ou après l'étape a), mettant éventuellement en oeuvre au moins un agent dispersant et/ou au moins un agent d'aide au broyage,
    c) au moins une étape de diminution de la concentration en ions OH- desdites dispersions et/ou desdites suspensions aqueuses, intervenant après l'étape a), à une valeur inférieure ou égale à $1\ x\ 10^{-2}$ mole/l,
    d) éventuellement au moins une étape d'addition d'au moins une substance à effet microbicide et/ou de mise en oeuvre d'un procédé physique de décontamination, intervenant avant pendant ou après l'étape a), et/ou b), et/ou c).

2.  Procédé selon la revendication 1 **caractérisé en ce que** la valeur de la concentration en ions OH- relative à l'étape a) est préférentiellement supérieure ou égale à 2 x $10^{-2}$ mole/l.

3. Procédé selon l'une des revendications 1 ou 2 **caractérisé en ce qu'**on réalise l'augmentation de la concentration en ions OH⁻, relative à l'étape a), à l'aide d'un ou plusieurs donneurs d'ions OH⁻, comme des oxydes alcalins et/ou alcalino-terreux et/ou des hydroxydes alcalins et/ou alcalino-terreux.

4. Procédé selon l'une des revendications 1 à 3 **caractérisé en ce que** la valeur de la concentration en ions OH⁻ relative à l'étape c) est préférentiellement inférieure ou égale à 1 x $10^{-3}$ mole/l et très préférentiellement inférieure ou égale à 1 x $10^{-4}$ mole/l.

5. Procédé selon l'une des revendications 1 à 4 **caractérisé en ce qu'**on réalise la diminution de la concentration en ions OH⁻, relative à l'étape c), à l'aide d'un ou plusieurs donneurs d'ions $H_3O^+$ faibles, moyennement forts ou forts, monovalents et/ou polyvalents, et préférentiellement à l'aide de $CO_2$ gazeux dissocié dans l'eau en acide carbonique.

6. Procédé selon l'une des revendications 1 à 5 **caractérisé en ce que** l'étape éventuelle d) d'addition d'au moins une substance à effet microbicide et/ou de mise en oeuvre d'un procédé physique de décontamination microbicide met en oeuvre au moins un biocide choisi parmi le *o*-phénylphénol et/ou ses sels ou bien leurs mélanges, et/ou parmi au moins un produit contenant un germe destructeur des germes microbiens, préférablement des germes pseudomonas, plus préférablement des germes pseudomonas aeruginosa, et **en ce que** ce germe destructeur est de la famille Bdellovibrio, et est très préférentiellement le germe Bdellovibrio bacteriovorus.

7. Procédé selon l'une des revendications 1 à 6 **caractérisé en ce que** l'étape éventuelle d) d'addition d'au moins une substance à effet microbicide et/ou de mise en oeuvre d'un procédé physique de décontamination microbicide met en oeuvre au moins un procédé de traitement basé sur l'augmentation de la température.

8. Procédé selon l'une des revendications 1 à 5 **caractérisé en ce qu'**il n'est pas mis en oeuvre d'agent microbicide selon l'étape d), et **en ce que** l'étape c) intervient préférentiellement entre une semaine et un mois après l'étape a).

9. Procédé selon l'une des revendications 1 à 8 **caractérisé en ce qu'**il est mis en oeuvre en discontinu, en semi-continu ou en continu.

10. Utilisation du procédé selon les revendications 1 à 9 pour désinfecter et/ou protéger des dispersions et/ou des suspensions aqueuses de matières minérales à traiter.

11. Procédé selon la revendication 1 **caractérisé en ce que** ces matières minérales sont choisies parmi des carbonates de calcium naturels et préférentiellement parmi le marbre, la calcite, la craie, la dolomite et leurs mélanges.

12. Procédé selon l'une des revendications 1 à 9 et 11 **caractérisé en ce qu'**il est mis en oeuvre dans les domaines de l'industrie minéralière, et notamment dans les réservoirs de stockage, les récipients de transport ferroviaire et routier, tels que les réservoirs en béton et en acier, les wagons citernes ferroviaires, les citernes et les conteneurs, dans l'industrie du papier, de préférence dans la fabrication du papier, et/ou dans le couchage du papier, ainsi que dans le domaine de la fabrication des peintures aqueuses et en outre dans des systèmes aqueux de peintures et vernis.

13. Procédé selon l'une des revendications 1 à 9, 11 et 12 **caractérisé en ce que** qu'il n'est pas mis en oeuvre de substances microbicides selon l'étape d).

**Claims**

1. A process for disinfection and/or preservation and/or reduction and/or control of microbial contamination of aqueous dispersions and/or suspensions of mineral matter chosen from among natural calcium carbonates and/or precipitated calcium carbonates and their mixtures, **characterized in that** it uses:

   a) at least one stage to increaser the OH⁻ concentration of said aqueous dispersions and/or said aqueous suspensions, to a value greater than or equal to 1 x $10^{-2}$ mole/l,
   b) at least one stage of dispersion and/or grinding of said aqueous dispersions and/or suspensions, occurring before, during or after stage a), possibly using at least on dispersing agent and/or at least one grinding aid agent,

c) at least one stage to reduce the OH$^-$ concentration of said aqueous dispersions and/or said aqueous suspensions, occurring after stage a), to a value less than or equal to 1 x 10$^{-2}$ mole/l,

d) possibly at least one stage of addition of at least one substance with a microbicidal effect and/or use of a physical decontamination process occurring before, during or after stage a), and/or stage b), and/or stage c).

2. A process according to claim 1, **characterized in that** the OH$^-$ concentration value relative to stage a) is preferentially higher than or equal 2x10$^{-2}$ mole/l.

3. A process according to one of claims 1 or 2 **characterized in that** the OH$^-$ concentration increase, relative to stage a), is undertaken using one or more OH$^-$ ion donators, such as alkaline and/or alkaline earth oxides and/or alkaline and/or alkaline earth hydroxides.

4. A process according to one of claims 1 to 3 **characterized in that** the OH$^-$ concentration value relative to stage c) is preferentially less or equal to 1x10$^{-3}$ mole/l, and very preferentially less than or equal to 1x10$^{-4}$ mole/l.

5. A process according to one of claims 1 to 4 **characterized in that** the OH$^-$ concentration reduction, relative to stage c), is undertaken using one or more weak, moderately strong or strong, monovalent and/or polyvalent H$_3$O$^+$ ion donators, and preferentially using gaseous CO2 dissociated into carbonic acid in water.

6. A process according to one of claims 1 to 5 **characterized in that** the possible stage d) of addition of at least one substance with a microbicidal effect and/or use of a physical process of microbicidal decontamination uses at least one biocide chosen from among o-phenlyphenol and/or its salts or their mixtures, and/or from among at least one product containing a germ which destroys microbial germs, preferably pseudomonas germs, more preferably pseudomonas aeruginosa germs, and **in that** the destructive germ is of the Bdellovibrio family, and is very preferably the Bdellovibrio bacteriovorous germ.

7. A process according to one of claims 1 to 6 **characterized in that** the possible stage d) of the addition of at least one substance with microbicidal effect and/or of use of a physical process of microbial decontamination uses at least one treatment process based on an increase in temperature.

8. A process according to one of claims 1 to 5 **characterized in that** no microbicidal agent is used according to stage d) and **in that** stage c) occurs preferentially between one week and one month after stage a).

9. A process according to one of claims 1 to 8 **characterised in that** it is used in discontinuous, semi-continuous or continuous way.

10. Use of a process according to one of claims 1 to 9 for disinfecting and/or preserving of dispersions and/or suspensions of aqueous mineral materials to be treated.

11. A process according to claim 1 **characterized in that** said mineral matter is chosen among natural calcium carbonates, preferably from among marble, calcite, chalk, dolomite and their mixtures.

12. A process according to one of claims 1 to 9 and 11 **characterized in that** it is used in the mineral industry fields, and notably in storage tanks, rail and road transport recipients, such as concrete and steel tanks, rail tanker wagons, tanks and containers, in the paper industry, preferably in paper manufacturing, and/or in coating colors, and in the field of the manufacture of water based paints as well as in lacquers and varnishes.

13. A process according to one of claims 1 to 9, 11 and 12 **characterized in that** no microbicidal substances are used in according to stage d).


**Patentansprüche**

1. Verfahren zur Desinfektion und/oder Konservierung und/oder Verringerung und/oder Kontrolle der mikrobiellen Kontamination von wässrigen Dispersionen und/oder wässrigen Suspensionen von Mineralstoffmaterialien, ausgewählt aus natürlichen und/oder gefällten Calciumcarbonaten und ihren Gemischen, **dadurch gekennzeichnet, dass** es verwendet:

a) wenigstens einen Schritt, bei dem die OH⁻-Ionenkonzentration der wässrigen Dispersionen und/oder der wässrigen Suspensionen auf einen Wert grösser oder gleich 1 x 10⁻² Mol/L erhöht wird,

b) wenigstens einen Schritt, bei dem die wässrigen Dispersionen und/oder die wässrigen Suspensionen dispergiert und/oder vermahlen werden, und der vor, während oder nach Schritt a) stattfindet, gegebenenfalls durch Verwenden wenigstens eines Dispergierungsmittels und/oder wenigstens eines Mahlhilfsmittels,

c) wenigstens einen Schritt, bei dem die OH⁻-Ionenkonzentration der wässrigen Dispersionen und/oder der wässrigen Suspensionen auf einen Wert kleiner oder gleich 1 x 10⁻² Mol/L vermindert wird, und der nach Schritt a) stattfindet,

d) gegebenenfalls wenigstens einen Schritt, bei dem wenigstens eine Substanz mit einem mikrobioziden Effekt zugegeben wird und/oder ein physikalisches Dekontaminierungsverfahren verwendet wird, und der vor, während oder nach Schritt a), und/oder b) und/oder c) stattfindet.

2. Verfahren nach Anspruch 1, **dadurch gekennzeichnet, dass** der Wert der OH⁻Ionenkonzentration bezüglich Schritt a) bevorzugt grösser oder gleich 2 x 10⁻² Mol/L ist.

3. Verfahren nach einem der Ansprüche 1 oder 2, **dadurch gekennzeichnet, dass** die Erhöhung der OH⁻-Ionenkonzentration, bezüglich Schritt a), mit Hilfe von einem oder mehreren OH⁻-Ionendonatoren, wie alkalischen Oxiden und/oder Erdalkalioxiden und/oder alkalischen Hydroxiden und/oder Erdalkalihydroxiden erfolgt.

4. Verfahren nach einem der Ansprüche 1 bis 3, **dadurch gekennzeichnet, dass** der Wert der OH⁻-Ionenkonzentration bezüglich Schritt c) bevorzugt kleiner oder gleich 1 x 10⁻³ Mol/L ist, und besonders bevorzugt kleiner oder gleich 1 x 10⁻⁴ Mol/L ist.

5. Verfahren nach einem der Ansprüche 1 bis 4, **dadurch gekennzeichnet, dass** die Verminderung der OH⁻-Ionenkonzentration, bezüglich Schritt c), mit Hilfe von einem oder mehreren schwachen, mittelstarken bis starken, monovalenten und/oder polyvalenten H₃O⁺-Ionendonatoren erfolgt, und bevorzugt mit Hilfe von gasförmigem CO₂, das in Wasser in Kohlensäure dissoziiert ist.

6. Verfahren nach einem der Ansprüche 1 bis 5, **dadurch gekennzeichnet, dass** der optionale Schritt d), bei dem wenigstens eine Substanz mit einem mikrobioziden Effekt zugegeben wird und/oder ein physikalisches Dekontaminierungsverfahren verwendet wird, wenigstens ein Biozid verwendet, das ausgewählt ist aus o-Phenylphenol und/oder seinen Salzen oder auch ihren Gemischen, und/oder aus wenigstens einem Produkt, das einen Keim enthält, der mikrobielle Keime zerstört, bevorzugt Pseudomonas Keime, und bevorzugter Pseudomonas Aeruginosa Keime, und dass der zerstörende Keim aus der Bdellovibrio Familie ist, und am meisten bevorzugt der Bdellovibrio Bacteriovorus Keim ist.

7. Verfahren nach einem der Ansprüche 1 bis 6, **dadurch gekennzeichnet, dass** der optionale Schritt d), bei dem wenigstens eine Substanz mit einem mikrobioziden Effekt zugegeben wird und/oder ein physikalisches Dekontaminierungsverfahren verwendet wird, wenigstens einen Behandlungsschritt verwendet, der auf einer Temperaturerhöhung basiert.

8. Verfahren nach einem der Ansprüche 1 bis 5, **dadurch gekennzeichnet, dass** in Schritt d) kein Mikrobiozid verwendet wird, und dass Schritt c) bevorzugt zwischen einer Woche und einem Monat nach Schritt a) erfolgt.

9. Verfahren nach einem der Ansprüche 1 bis 8, **dadurch gekennzeichnet, dass** es diskontinuierlich, semi-kontinuierlich oder kontinuierlich durchgeführt wird.

10. Verwendung eines Verfahrens gemäss einem der Ansprüche 1 bis 9 zur Desinfektion und/oder zum Schutz der zu behandelnden wässrigen Dispersionen und/oder wässrigen Suspensionen von Mineralstoffmaterialien.

11. Verfahren nach Anspruch 1, **dadurch gekennzeichnet, dass** die Mineralstoffmaterialien ausgewählt sind aus natürlichen Calciumcarbonaten und bevorzugt aus Marmor, Calcit, Kreide, Dolomit und ihren Gemischen.

12. Verfahren nach einem der Ansprüche 1 bis 9 und 11, **dadurch gekennzeichnet, dass** es auf dem Gebiet der Mineralstoffindustrie, und insbesondere in Lagertanks, Bahn- und Strassentransportbehältern, wie Beton- und Stahltanks, Bahntankwaggons, Tanks und Behältern, in der Papierindustrie, bevorzugt bei der Papierherstellung und/oder beim Streichen von Papier, sowie auf dem Gebiet der Herstellung von wasserbasierten Farben und zudem wässrigen Systemen von Farben und Lacken verwendet wird.

**13.** Verfahren nach einem der Ansprüche 1 bis 9, 11 und 12, **dadurch gekennzeichnet, dass** es in Schritt d) keinen mikrobioziden Substanzen verwendet.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 10027588 A1
- WO 0490148 A
- CN 1483773
- JP 2004169243 B
- DE 19811742
- EP 1149172 A

**Littérature non-brevet citée dans la description**

- Kunststoffe im Lebensmittelverkehr. Carl Heymanns Verlag, 01 Avril 2001
- **KARL HEINZ WALLHÄUSSER.** Praxis der Sterilisation, Desinfektion-Konservierung. Georg Thieme Verlag
- **WILFRIED PAULUS.** Microbicides for the protection of materials, a handbook. Chapman & Hall, 1993
- *Code of Federal Regulation 21,* Avril 2001
- **KARL-HEINZ WALLHÄUSSER.** Praxis der Sterilisation, Desinfektion-Konservierung. Georg Thieme Verlag, 1995, 43
- **H. WEBER.** Wörterbuch der Mikrobiologie. Gustav Fischer Verlag, 1997, 449, 321
- High Electric Field Puls. bulletin ASE/AES 3/01. Mars 2001, 44
- *J. Food Prot.,* 2001, vol. 64 (10), 1579-1583
- **MADIGAN, M.T. ; MARTINKO, J.M. ; PERKER, J.** Brock-Biology of Microorganisms. Prentice-Hall, Inc, 2000, 154-155
- Allgemeine Mikrobiologie. Georg Thieme Verlag, 1992, 194-196
- Bestimmung von aeroben Bakterien und Keime. 1985
- Bestimmung von Pilzen. 1985
- Bestimmung von aeroben mesophilen Keimen. Schweizerisches Lebensmittelbuch. 1985
- Standard Methods for the Examination of Dairy Products. American Public Health Association, 1985
- American Water Works Association and Water Pollution Control Federation : Standard Methods for the Examination of Water and Wastewater. American Public Health Association, 1998
- An improved agar medium for the detection of proteolytic organisms in total bacterial counts. *J. Appl. Bact.,* 1970, vol. 33, 363-370